(19) **Europäisches Patentamt**
**European Patent Office**
**Office européen des brevets**

(11) **EP 2 821 008 A1**

(12) **EUROPEAN PATENT APPLICATION**
published in accordance with Art. 153(4) EPC

(43) Date of publication:
**07.01.2015 Bulletin 2015/02**

(21) Application number: **13755448.1**

(22) Date of filing: **14.02.2013**

(51) Int Cl.:
**A61B 5/0245** (2006.01)   **A61B 5/1455** (2006.01)

(86) International application number:
**PCT/JP2013/000807**

(87) International publication number:
**WO 2013/128825 (06.09.2013 Gazette 2013/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.02.2012   JP 2012040994**

(71) Applicant: **Konica Minolta, Inc.**
**Tokyo 100-7015 (JP)**

(72) Inventor: **HAMAGURI, Kenji**
**Chiyoda-ku**
**Tokyo 100-7015 (JP)**

(74) Representative: **Hoffmann Eitle**
**Patent- und Rechtsanwälte PartmbB**
**Arabellastraße 30**
**81925 München (DE)**

(54) **BIOINFORMATION PROCESSING APPARATUS AND SIGNAL PROCESSING METHOD**

(57)    Disclosed is a biological information processing apparatus and a signal processing method, wherein a biological signal comprising a first signal component having periodicity is generated, and a given frequency distribution is generated based on a second-order difference signal obtained by subjecting the biological signal to a second-order differencing operation, whereafter, with respect to the generated frequency distribution, an effective greatest frequency zone which is a zone having a greatest frequency of an interval-time is determined based on a given criterion, and a period of the first signal component is calculated based on an average time interval in the effective greatest frequency zone.

FIG. 2

**Description**

Technical Field

The present invention relates to a biological information processing apparatus and a biological information processing method capable of removing a noise component from a time-series signal.

Background Art

Heretofore, a technique concerning a signal processing of removing from time-series data a noise component superimposed thereon has been applied to various signal processing apparatuses. In particular, when time-series data includes information about biological information, the signal processing apparatus is called "biological information measuring apparatus". This biological information measuring apparatus is an apparatus configured to detect biological information from a biological tissue in a non-invasive manner, and specific examples of the biological information measuring apparatus include: a measuring apparatus configured to measure a pulse wave pattern and a pulse rate of a living body, called "photoelectric pulse wave meter", and a measuring apparatus configured to measure arterial oxygen saturation, called "pulse oxymeter". A principle of the measuring apparatuses is to derive biological information, such as a concentration of a light-absorbing substance in blood, based on a signal component corresponding to fluctuation due to pulsation of the biological tissue, which is obtainable by receiving light transmitted through or reflected by a biological tissue.

Generally, on data obtained by receiving light transmitted through or reflected by a biological tissue, as data required for detecting biological information, various noise components are superimposed. The noise components mostly arises from body movement, for example, when a living body moves during use of a biological information measuring apparatus. Because a noise component superimposed on the signal component becomes a factor causing error in calculating biological information, it is desired to remove the noise component.

There has been proposed a technique of emitting a plurality of light beams having respective different wavelengths to a living body, and calculating biological information, based on a DC-AC ratio of an intensity of each of corresponding light beams transmitted through or reflected by a biological tissue. Particularly, in the case where a noise component is superimposed on the signal component, a DC-AC ratio in regard to each of the wavelengths is represented by the signal component and the noise component. As means to remove the noise component represented in this manner, there has been proposed a technique of extracting, from data generated based on measured data and including a signal component having periodicity, the signal component by use of the periodicity, thereby removing a noise component from the data (see, for example, the following Patent Literature 1).

This technique is capable of measuring arterial oxygen saturation and pulse rate in a non-invasive manner, without occurrence of an error due to body movement.

However, the above technique is intended to remove noise due to fluctuation of venous blood occurring, for example, when a person or user moves his/her finger or hand to which a probe is attached, so that it has difficulty in removing noise due to fluctuation of arterial blood occurring, for example, when a person or user walks while moving his/her entire arms.

Thus, the technique disclosed in the Patent Literature 1 is incapable of sufficiently removing noise superimposed on a pulse wave signal, during walking or the like, so that an error occurs in a measured value of pulse rate during walking or the like.

Citation List

Patent Literature

Patent Literature 1: WO 2010/073908A

Summary of Invention

The present invention has been made in view of the above circumstances, and an object thereof is to provide a technique of removing a noise component superimposed on a pulse wave signal during walking or the like to thereby more accurately measure pulse rate.

In a biological information processing apparatus and a signal processing method of the present invention, first measurement data comprising a first signal component having periodicity and a first noise component, and the second measurement data comprising a second signal component having a given first relationship with the first signal component and a second noise component having a given second relationship with the first noise component, are measured, and a biological signal including the first signal component is generated based on an estimate of a given third relationship, the first measurement data and the second measurement data. Then, a given frequency distribution is generated based on a second-order difference signal obtained by subjecting the biological signal to a second-order differencing operation. With respect to the generated frequency distribution, an effective greatest frequency zone which is a zone having a greatest frequency of the interval-time is determined based on a given criterion, and a period of the first signal component is calculated based on an average time interval in the effective greatest frequency zone. As above, the biological information processing apparatus and the signal processing method of the present invention are an apparatus and method using a new technique for removing a noise component to make it possible to remove a noise component superimposed on a pulse wave signal during walking or the like, thereby more accurately measuring pulse rate.

These and other objects, features, and advantages of the present invention will become apparent upon reading of the following detailed description along with the accompanying drawings.

Brief Description of Drawings

FIG. 1 is a block diagram illustrating a configuration of a biological information processing apparatus according to one embodiment.
FIG. 2 is a block diagram illustrating a configuration of a pulse rate calculation section in the biological information processing apparatus.
FIG. 3 is a diagram illustrating each example of a second-order difference signal, and inter-peak, inter-valley and inter-rising intervals, in the biological information processing apparatus.
FIG. 4 is a diagram illustrating one example of a peak frequency distribution, in the biological information processing apparatus.
FIG. 5 is a diagram for explaining a weight for use in creating the peak frequency distribution, in the biological information processing apparatus.
FIG. 6 is a diagram illustrating one example of a valley frequency distribution, in the biological information processing apparatus.
FIG. 7 is a diagram for explaining a weight for use in creating the valley frequency distribution, in the biological information processing apparatus.
FIG. 8 is a diagram illustrating one example of a rising frequency distribution, in the biological information processing apparatus.
FIG. 9 is a diagram for explaining a weight for use in creating the rising frequency distribution, in the biological information processing apparatus.
FIG. 10 is a flowchart illustrating a processing of determining an effective greatest frequency zone and an effective greatest weighted-frequency zone, in the biological information processing apparatus.
FIG. 11 is a flowchart illustrating processing steps S20 to S26 of a period calculation process, in the biological information processing apparatus.
FIG. 12 is a flowchart illustrating processing steps S27 to S34 of the period calculation process, in the biological information processing apparatus.
FIG. 13 is a flowchart illustrating a pulse rate calculation process, in the biological information processing apparatus.
FIG. 14 is a flowchart illustrating an oxygen saturation and pulse rate measurement process, in the biological information processing apparatus.
FIG. 15 is a diagram illustrating one example of a result of pulse rate measurement, in the biological information processing apparatus and a comparative apparatus.

Description of Embodiments

A principle of the present invention will first be described, and then one embodiment of the present invention will be described based on the drawings. Elements or components assigned with the same reference sign in the figures means that they are identical, and therefore duplicated description thereof will be omitted appropriately. In this specification, when a term collectively means a plurality of identical elements or components, it is designated by a reference sign without any suffix, whereas, when the term means a specific one of the elements or components, it is designated by the reference sign with a suffix.

< Principle of present invention >

The following description will be made on an assumption that, based on a plurality of measurement data obtained by emitting a plurality of light beams having respective different wavelengths to a living body and receiving corresponding light beams transmitted through or reflected by the living body, pulse rate and blood oxygen saturation are measured as biological information of the living body.

According to a so-called Beer-Lambert law, it is approximated that a ratio between an AC component and a DC component of an intensity of light having a certain wavelength after being transmitted through or reflected by a biological tissue is equivalent to a variation of absorbance of the biological tissue at the wavelength.

From the use of the approximation based on the Beer-Lambert law, time-series data IR_signal about an infrared DC-AC ratio which is a ratio between a DC component and an AC component of an intensity of transmitted light or reflected light, in regard to an infrared wavelength IR, can be deemed to be equivalent to a variation of absorbance of a biological tissue in regard to the infrared wavelength IR. Similarly, time-series data R_signal about a red DC-AC ratio which is a ratio between a DC component and an AC component of an intensity of transmitted light or reflected light, in regard to a red wavelength R, can be deemed to be equivalent to a variation of absorbance of a biological tissue in regard to the red wavelength R.

The time-series data IR_signal about the infrared DC-AC ratio is expressed by the following formula (1):

$$\text{IR\_signal} = s + n \qquad \text{--- (1)}$$

In this formula, s is a signal component corresponding to the variation of absorbance, and n is a noise component superimposed on the signal component.

The time-series data R_signal about the red DC-AC ratio is expressed by the following formula (2):

$$\text{R\_signal} = s \times k\_a + n \times k\_v \qquad \text{--- (2)}$$

In this formula, k_a is a ratio between the signal component s corresponding to a variation of absorbance in light having the wavelength IR and a signal component corresponding to a variation of absorbance in light having the wavelength R, and k_v is a ratio between the noise component n superimposed on the signal component in regard to the infrared wavelength IR and a noise component superimposed on the signal component in regard to the red wavelength R.

The k_a in the formula (2) is equivalent to a ratio of an absorption coefficient of light with the red wavelength R to an absorption coefficient of light with the infrared wavelength IR, in arterial blood, wherein it is known that the k_a has one-to-one correspondence with respect to arterial oxygen saturation. Thus, the arterial oxygen saturation can be derived by calculating the k_a.

Further, the following formula (3) can be obtained by multiplying the formula (1) by the k_v, and the following formula (4) can be obtained by subtracting the formula (2) from the formula (3):

$$\text{IR\_signal} \times k\_v = s \times k\_v + n \times k\_v \qquad \text{--- (3)}$$

$$\text{IR\_signal} \times k\_v - \text{R\_signal} = s \times (k\_v - k\_a) \qquad \text{--- (4)}$$

Similarly, the following formula (5) can be obtained by multiplying the formula (1) by the k_a, and the following formula (6) can be obtained by subtracting the formula (2) from the formula (5):

$$\text{IR\_signal} \times k\_a = s \times k\_a + n \times k\_a \qquad \text{--- (5)}$$

$$\text{IR\_signal} \times k\_a - \text{R\_signal} = n \times (k\_a - k\_v) \qquad \text{--- (6)}$$

Then, by using a relationship that the signal component s and the noise component n are independent of each other, i.e., the following relational formula (7), and under a condition that each of the k_a and k_v is constant within a short period of time, a correlation between the formula (4) and the formula (6) is found to thereby obtain the following formula (8):

$$\Sigma_i \, (s \times n) = 0 \qquad\qquad \text{--- (7)}$$

$$\Sigma_i \, \{IR\_signal \times k\_v - R\_signal\} \times \{IR\_signal \times k\_a - R\_signal\}$$

$$= \{(k\_v - k\_a\} \times (k\_a - k\_v\} \times \Sigma_i \, (s \times n) \qquad\qquad \text{--- (8)}$$

$$= 0$$

In these formulas, $\Sigma$ is a sum within a short period of time enough to satisfy the condition that each of the k_a and k_v is constant. Further, i is a data number of the time-series data IR_signal and the time-series data R_signal indicative of a variation of light intensity, wherein the i is associated with a time t in the following relationship: $t = \Delta t \times i + t0$, where $\Delta t$ is a data measurement time interval, and t0 is a measurement start clock time.

The formula (8) includes two unknowns: k_v and k_a, and therefore it is impossible to derive the k_v and k_a only from the formula (8).

Therefore, in order to derive the k_v, the formula (4) is used, wherein a right-hand side of the formula (4) is an approximately periodic (or cyclic) value, so that the k_v is derived as a value allowing a left-hand side of the formula (4) to have periodicity. In case of noise due to usual body movement, the k_v can be deemed as a value pv corresponding to a ratio between an absorption coefficient in regard to the wavelength R and an absorption coefficient in regard to the wavelength IR, in venous blood, so that the k_v is used as an estimate pv_es of the pv.

A value of the k_v derived in the above manner is assigned to the formula (8), and the k_a satisfying the formula (8) is derived. Then, the derived value of the k_a is used as an estimate pa_es of a value pa corresponding to the ratio between the absorption coefficient in regard to the wavelength R and the absorption coefficient in regard to the wavelength IR, in arterial blood (arterial blood absorption coefficient ratio pa). Based on this pa_es, the arterial oxygen saturation can be derived while reducing a noise component.

Pulse rate can be derived from the derived k_v, R_signal and IR_signal. More specifically, the following formula (9) (equivalent to the formula (4)) is established, and thus an average value of periods within a given time in the [R(i) - k_v $\times$ IR(i)] is calculated:

$$R(i) - k\_v \times IR(i) \fallingdotseq (k\_a - k\_v) \times s(i) \qquad\qquad \text{--- (9)}$$

Then, pulse rate is derived as a reciprocal of the period.

< Embodiment >

In the case where noise superimposed on a pulse wave signal is caused by fluctuation in flow rate of venous blood, arterial oxygen saturation and pulse rate can be calculated by the method described in the principle of the present invention. That is, the noise due to fluctuation in flow rate of venous blood can be almost completely removed.

However, noise during body movement such as walking is not dominantly caused by fluctuation in flow rate of venous blood, but largely caused by fluctuation in flow rate of blood having a value of oxygen saturation which is intermediate between those of arterial blood and venous blood, so that the k_v has a value close to that of the k_a. Thus, during walking or the like, if a value close to the venous blood absorption coefficient ratio pv is assigned to the k_v, the [R(i) - k_v $\times$ IR(i)] does not have high periodicity, and noise largely remains in a signal represented by the formula (9). Therefore, even if a period is derived, it is impossible to derive a correct period and thus it is impossible to accurately calculate pulse rate. The venous blood absorption coefficient ratio pv means a ratio between an absorption coefficient of light with the wavelength R and an absorption coefficient of light with the wavelength IR, in venous blood, as mentioned above.

In the case where the k_v has a value close to that of the k_a, the formula (8) is expressed as follows:

$$k\_a = \{k\_v \times \Sigma R(i) \times IR(i) - \Sigma R(i)^2\} \, / \, \{k\_v \times \Sigma IR(i)^2 - \Sigma IR(i) \times R(i)\} \fallingdotseq k\_a$$

Therefore, the k_a can be approximately accurately calculated, i.e., the arterial oxygen saturation can be approximately accurately calculated. Thus, a value of the k_a calculated from the formula (8) is used as the pa_es. From a physiological relationship between the pa_es and the venous blood absorption coefficient ratio pv corresponding to the venous oxygen saturation, the pv is estimated and used as the pv_es. In the case where noise is caused by fluctuation in venous blood, the k_v is calculated on an assumption that it is equal to the pv, as mentioned above.

On a pulse wave signal during waking or the like, noise due to fluctuation of blood having a value of oxygen saturation which is intermediate between those of arterial blood and venous blood is superimposed. Thus, in this embodiment, an intermediate value p between the estimate pa_es of the pa corresponding to the arterial blood absorption coefficient ratio and the estimate pv_es of the pv corresponding to the venous blood absorption coefficient ratio is used to thereby reduce noise in the [R(i) - p × IR(i)] indicated in the formula (9).

Further, a wave period is calculated by using a second-order difference waveform of the [R(i) - p × IR(i)], and the pulse rate is derived with less error.

< Method of calculating pulse rate >

In this embodiment, an intermediate value p between the estimate pa_es of the pa corresponding to the arterial blood absorption coefficient ratio and the estimate pv_es of the pv corresponding to the venous blood absorption coefficient ratio is used as the k_v to reduce noise in the [R(i) - k_v × IR(i)] indicated in the formula (9), and a second-order difference waveform of the [R(i) - k_v × IR(i)] is used to calculate a wave period. Thus, this embodiment will be described based on an example in which a wave period is calculated three times by using the p set to different values, and a period in this measurement is determined by using the three estimated periods and a previously determined period, to thereby derive pulse rate. A value of the p is set based on the estimate pa_es for arterial blood or the estimate pv_es for venous blood and from a physiological relationship therebetween and heuristics.

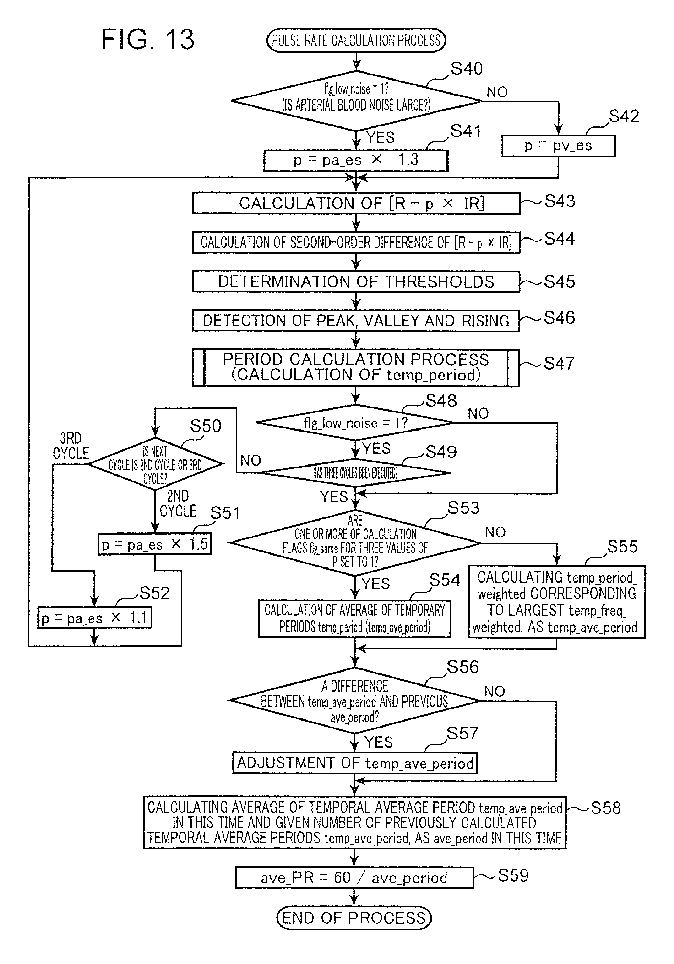

FIG. 13 is a flowchart illustrating a pulse rate calculation process.

First of all, it is determined whether or not noise due to fluctuation of the aforementioned blood having a value of oxygen saturation close to a value of arterial oxygen saturation (this noise will hereinafter be referred to as "arterial blood noise") is large. Specifically, a noise index is calculated using the following formula (10), and the above determination is performed.

$$\text{Noise index} = [\Sigma\Delta R(t)^2 - pa\_es \times \{\Sigma 2 \times \Delta R(t) \times \Delta IR(t) - pa\_es \times \Sigma\Delta IR(t)^2\}] / [\Sigma\Delta R(t)^2 - pv\_es$$

$$\times \{\Sigma 2 \times \Delta R(t) \times \Delta IR(t) - pv\_es \times \Sigma\Delta IR(t)^2\}] \qquad \text{--- (10)}$$

In this formula, IR and R represent, respectively, IR_signal and R_signal, and $\Delta IR$ and $\Delta R$ represent, respectively, a temporal difference in IR(t) and a temporal difference in R(t). Further, t is a serial index for N measurement data being continuously measured. $\Sigma$ is a sum calculated by setting the t in the range of 1 to N, and the serial index t denotes t-th measurement data among the N measurement data. In this formula, the temporal differences $\Delta IR$, $\Delta R$ are used. Alternatively, IR(t) and R(t) may be used.

When the noise index is less than a threshold, flg_low_noise is set to 1 (flg_low_noise ← 1), otherwise (when the noise index is equal to or greater than the threshold), the flg_low_noise is set to 0 (flg_low_noise ← 0).

When the noise index is less than the threshold (flg_low_noise = 1), i.e., the arterial blood noise is large (Step S40: YES), processing in Steps S43 to S47 will be repeatedly performed by using, as a value of the p, the following three values: pa_es × 1.3; pa_es × 1.5; and pa_es × 1.1. Thus, the pa_es × 1.3 is first set as the value of the p (Step S41). On the other hand, when the arterial blood noise is small (flg_low_noise = 0), i.e., (Step S40: NO), the estimate pv_es for venous blood is set as the value of the p (Step S42), and the processing in Steps S43 to S47 are executed only once. In this routine, when the flg_low_noise = 1, the values pa_es × 1.3, pa_es × 1.5 and pa_es × 1.1 are used as the p. Alternatively, pa_es + $\Delta$p1, pa_es + $\Delta$p2 and pa_es + $\Delta$p3 may be used, where $\Delta$p1, $\Delta$p2 and $\Delta$p3 are, respectively, different positive values appropriate to the pa_es or different positive values appropriate to the oxygen saturation SpO2. Alternatively, pv_es - $\Delta$p1, pv_es - $\Delta$p2 and pv_es - $\Delta$p3 may be used, where $\Delta$p1, $\Delta$p2 and $\Delta$p3 are, respectively, different positive values appropriate to the pa_es or different positive values appropriate to the oxygen saturation SpO2. Further, the number of the p is not necessarily set to three, but may be set to one or may be set to three or more.

When the p is set, a signal of the [R(i) - p × IR(i)] indicated in the formula (9) is calculated using the set value of the p (Step S43). Specifically, p_pulse(i) = R(i) - p × IR(i) is calculated from an R pulse waveform R(i) and an IR pulse waveform IR(i) each having a duration of past T seconds from a current time. The i is a data number of the time-series data IR_signal and the time-series data R_signal indicative of a variation of light intensity, i.e., the i is a serial index of 0 to N - 1.

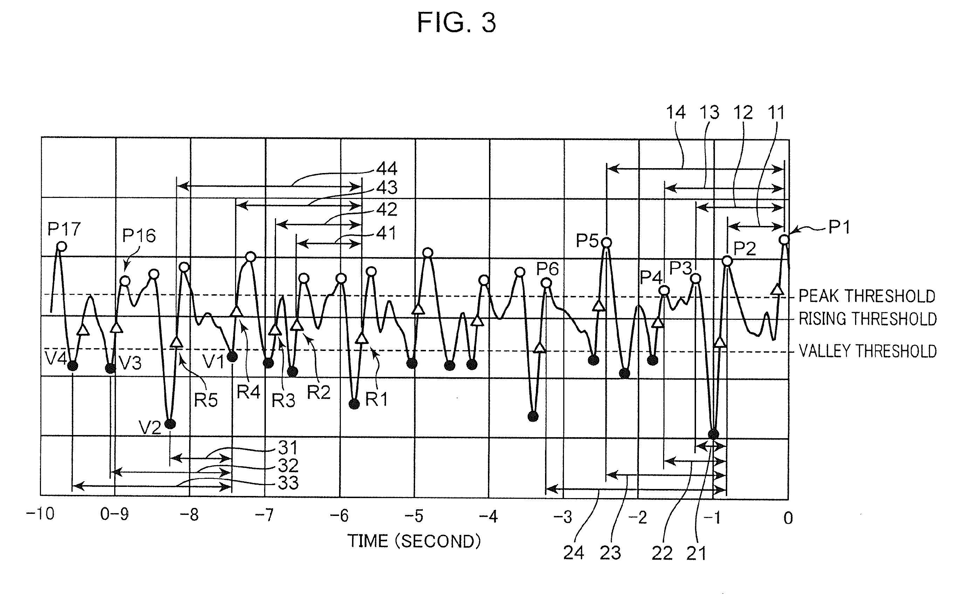

Then, the p_pulse(i) calculated in the Step S43 is moving-averaged, and a second-order difference p_pulse_2(i) of a result of the moving average is calculated (Step S44). This second-order difference p_pulse_2(i) will be referred to as "second-order difference signal". FIG. 3 illustrates one example of the second-order difference signal.

In order to detect a peak and a valley from the second-order difference signal, a peak threshold and a valley threshold are calculated (Step S45). Specifically, the greatest peak value, the second-greatest peak value and the third-greatest peak value are retrieved from the second-order difference signal, and the peak threshold is determined based on these values. In cases where there is no need to reduce an amount of calculation, the peak threshold may be determined from a root-mean-square value of the second-order difference signal. If the peak value is determined based on the greatest peak value, an infrequently-occurring large noise has a strong influence on the setting. Thus, when a ratio between the greatest peak value and the second-greatest peak value is greater than a predetermined value, a value obtained by multiplying an average value of the second-greatest and third-greatest peak values by a given coefficient is used as the threshold. On the other hand, in the case where the ratio between the greatest peak value and the second-greatest peak value is equal to or less than the predetermined value, when a ratio between the greatest peak value and the third-greatest peak value is greater than a predetermined value, a value obtained by multiplying the third-greatest peak value by a given coefficient is used as the threshold, and, when a ratio between the greatest peak value and the third-greatest peak value is equal to or less than the predetermined value, a value obtained by multiplying an average value of the greatest, second-greatest and third-greatest peak values by a given coefficient is used as the threshold. Similarly, the deepest, second-deepest and third-deepest valley values are retrieved, and the valley threshold is determined based on these values. Then, a rising threshold is determined based on the peak threshold and the valley threshold. For example, (peak threshold - valley threshold), i.e.. a value obtained by subtracting the valley threshold from the peak threshold is used as the rising threshold.

Then, effective peak, valley and rising are detected from the second-order difference signal (Step S46). Specifically, peaks each having a peak value greater than the peak threshold are retrieved (see P1 to P17 in FIG. 3), and a peak interval falling within a predetermined interval (e.g., within a range of period corresponding to a pulse rate of 20 bpm to 250 bpm) is evaluated as an effective peak interval (see intervals 11 to 14 in FIG. 3). A predetermined number of the effective peak intervals are set for each of the peaks greater than the peak threshold. Similarly, valleys each having a valley value less than the valley threshold are retrieved (see V1 to P4 in FIG. 3), and a valley interval falling within a predetermined interval is evaluated as an effective valley interval and set as a valley interval (see intervals 31 to 33 in FIG. 3). Further, risings are detected from the second-order difference signal, and, when the (peak threshold - valley threshold), i.e., a value obtained by subtracting the valley threshold from the peak threshold, corresponding to one of the detected risings (see R1 to R5 in FIG. 3), exceeds the rising threshold, an interval between the one rising and an adjacent rising exceeding the rising threshold is calculated. When the calculated intervals falls within a predetermined value, it is evaluated as an effective rising interval and set as a rising interval (see intervals 41 to 44 in FIG. 3).

The peak interval, the valley interval and the rising interval will be described in more detail below. Each of these intervals, for example, the peak interval includes not only an interval with respect to an adjacent peak, but also an interval with respect to second-adjacent peak and others.

The more detailed description will be made with reference to FIG. 3. In the second-order difference signal illustrated in FIG. 3, the white circle mark (○), the black circle mark (●) and the triangle mark (△) denote a peak, a valley and a rising, respectively. Only a peak exceeding the preset peak threshold and only a valley below the preset valley threshold are used for measurement. Further, a rising in which (peak height - valley depth) of a valley and a peak following immediately after the valley is greater than the rising threshold based on the peak threshold and the valley threshold, e.g., the (peak threshold - valley threshold), is detected, and, for example, an average of a clock time of the peak and a clock time of the valley is calculated as a rising time.

First of all, intervals the peak P1 are measured. More specifically, the interval 11 between the peak P1 and the peak P2 is measured. Further, the interval 12 between the peak P1 and the peak P3, the interval 13 between the peak P1 and the peak P4 and the interval 14 between the peak P1 and the peak P5 are measured. In this embodiment, four intervals are measured on the basis of each peak. Similarly, intervals on the basis of the peak P2, i.e., intervals 21, 22, 23, 24, are measured. In this manner, intervals will be measured until measurement for the last peak 16 is completed. In this case, the number of intervals on the basis of the peak P16 is one with respect to the peak 17.

Similarly, intervals on the basis of each valley and each rising are measured. It should be noted that, in FIG. 3, a reference sign is assigned to only a part of valleys and risings, for simplicity of illustration. Regarding intervals on the basis of each valley, for example, the valley V1, the interval 31 between the valley V1 and the valley V2, the interval 32 between the valley V1 and the valley V3 and the interval 33 between the valley V1 and the valley V4 are measured. Regarding intervals on the basis of each rising, for example, the rising R1, the interval 41 between the rising R1 and the rising R2, the interval 42 between the rising R1 and the rising R3, the interval 43 between the rising R1 and the rising R4 and the interval 44 between the rising R1 and the rising R5 are measured. In this embodiment, only intervals between the rising points are measured. Alternatively, intervals between falling points may be measured, in place of or in addition to intervals between the rising points. Further, in this embodiment, for each of all of the peaks (valleys or risings), intervals with respect to a plurality of adjacent peaks (valleys or risings) are measured. However, it is not essential to perform the measurement for all of the peaks (valleys or risings), and measure the same number of intervals on the basis of each peak (valley or rising).

After completion of the measurement of three types of intervals: the peak intervals; the valley intervals; and the rising intervals, a period calculation process in Step S47 in FIG. 13 is executed.

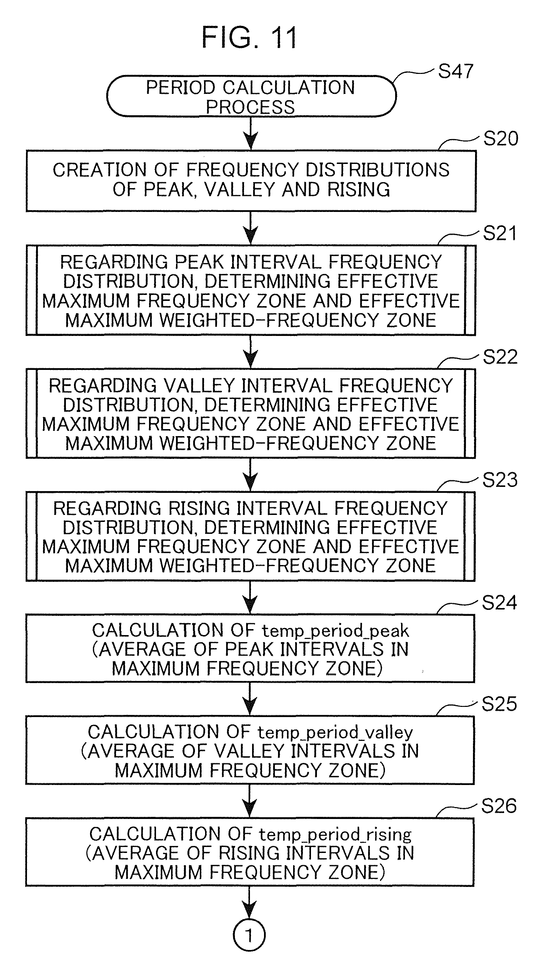

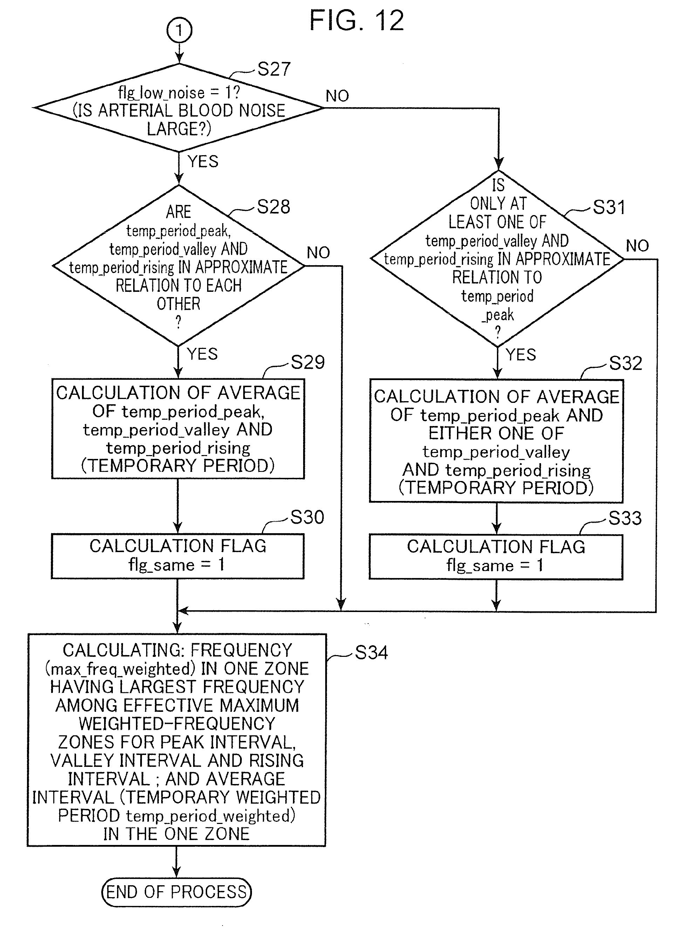

With reference to FIGS. 11 and 12, details of the period calculation process in Step S47 will be described below.

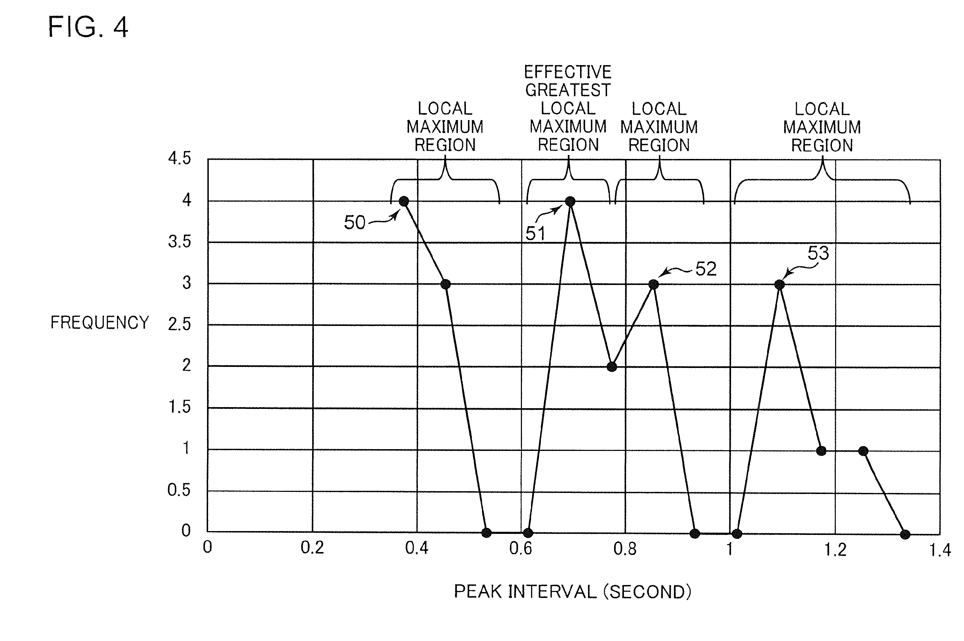

In the period calculation process, first of all, with respect to each of the group of peak interval, the group of valley interval and the group of rising interval, a frequency distribution (frequency distribution table) is created (Step S20). FIG. 4 illustrates one example of a peak frequency distribution. The horizontal axis represents measured interval-time, and the vertical axis represents frequency. The frequency distribution is created by sorting the measured intervals into a plurality of zones each having a predetermined given time width (the zone will hereinafter be referred to as "class interval"), and calculating the number of the intervals in each class. Then, the class intervals are set on the horizontal axis, and the number of the intervals in each class interval is plotted with respect to the vertical axis as a frequency. For example, the classes may be set to have a width of 0.01 sec to less than 0.02 sec, a width of 0.02 sec to less than 0.03 sec.

From each of the three types of created frequency distribution tables, an effective greatest frequency zone and an effective greatest weighted-frequency zone are determined (Step S21 to Step S23). The determination of the effective greatest frequency zone and the effective greatest weighted-frequency zone will be described in detail in the subsequent section < Method of determining effective greatest frequency zone and effective greatest weighted-frequency zone >

The term "effective greatest frequency zone" means a zone which is assumed to be usable for determining a period with highest reliability. The term "effective greatest weighted-frequency zone" means a zone which is estimated to be usable for determining a period with highest reliability, in a frequency distribution after subjecting frequencies in the frequency zones to weighting based on a previously-calculated period.

Subsequently, a period temp_period_peak is derived by dividing a sum of the peak intervals in the zone by a sum of the frequencies in the effective greatest frequency zone for peak (Step S24). Similarly, a period temp_period_valley is derived by dividing a sum of the valley intervals in the zone by a sum of the frequencies in the effective greatest frequency zone for valley (Step S25), and a period temp_period_rising is derived by dividing a sum of the rising intervals in the zone by a sum of the frequencies in the effective greatest frequency zone for rising (Step S26),

Subsequently, a temporary period temp_period is determined. How to calculate the temporary period temp_period varies depending on a level of the arterial blood noise.

When the flg_low_noise = 1, i.e., the arterial blood noise is large (Step S27 in FIG. 12: YES), and only in a situation where the average peak interval temp_period_peak, the average valley interval temp_period_valley and the average setting interval temp_period_rising derived in the Steps S24 to S26 are in approximate relation to each other (Step S28: YES), the temporary period temp_period is calculated (Step S29). Specifically, (temp_period_valley / temp_period_peak) and (temp_period_rising / temp_period_peak) are calculated. Then, when both of them fall within the range of 1 - $\alpha$ to 1 + $\alpha$, an average of the average peak interval temp_period_peak, the average valley interval temp_period_valley and the average setting interval temp_period_rising is calculated, and the calculated average is used as the temporary period temp_period. The $\alpha$ is a setting value for determining a level of approximation. For example, it is a numerical value of about 0.05, and preliminarily set. Then, a calculation flag flg_same is set to "1" indicating that the temporary period temp_period has been calculated (Step S30). When the three periods are not in approximate relation (the Step S28: NO), no temporary period temp_period has been calculated, and the calculation flag flg_same is maintained in a state in which it is set to "0" indicating that no temporary period temp_period has been calculated (flg_same = 0).

On the other hand, when the arterial blood noise is small (flg_low_noise = 0) (the Step S27: NO), and only in a situation where at lest one of the average valley interval temp_period_valley and the average setting interval temp_period_rising is in approximate relation to the average peak interval temp_period_peak (Step S3 1: YES), the temporary period temp_period is calculated (Step S32). Specifically, (temp_period_valley / temp_period_peak) and (temp_period_rising / temp_period_peak) are calculated. Then, when at least one of them falls within the range of 1 - $\alpha$ to 1 + $\alpha$, an average of the two periods thereof is calculated, and the calculated average is used as the temporary period temp_period. Then, the calculation flag flg_same is set to "1" indicating that the temporary period temp_period has been calculated (flg_same = 1) (Step S33). When the three periods are not in approximate relation (the Step S31: NO), no temporary period temp_period has been calculated, and the calculation flag flg_same is maintained in a state in which it is set to "0" indicating that no temporary period temp_period has been calculated (flg_same = 0).

Subsequently, an average interval in the effective greatest weighted-frequency zones derived in the Steps S21 to S23. Among the effective greatest weighted-frequency zones for peak, valley and rising, one effective greatest weighted-frequency zone having a greatest one of the frequencies of the zones, is identified, and a temporary weighted period temp_period_weighted is derived by dividing a sum of the intervals in the identified effective greatest weighted-frequency zone by the frequency in the zone (Step S34).

As mentioned above, when flg_low_noise = 1, the period calculation process is performed three times while changing the value of the p. Thus, with respect to each of the values of the p, the temporary periods temp_period and the temporary weighted periods temp_period_weighted are calculated, and three calculation flags flg_same are set, correspondingly to the values of the p. When flg_low_noise = 0, the period calculation process is performed only once while setting the p to the pv_es (p = pv_es), as mentioned above. Thus, only one temporary period temp_period and only one temporary weighted period temp_period_weighted are calculated, and only one calculation flag flg_same is set.

Returning to FIG. 13, processing after the period calculation process will be described. Following the period calculation process, it is first checked whether or not the flg_low_noise is 1 (Step S48).

When flg_low_noise is 1 (the Step S48: YES), it is checked whether or not the processing in the Step S43 to the Step S47 have been performed for all of the three values of the p (Step S49). When each of the temporary period temp_period and the temporary weighted period temp_period_weighted has not been calculated three times (the Step S49: NO), and the next processing cycle is a 2nd cycle (Step S50: 2nd cycle), the pa_ex $\times$ 1.5 is set as the p (Step S51), and the processing in the Step S43 to the Step S47 are performed to calculate second values of the temporary period temp_period and the temporary weighted period temp_period_weighted. Differently, when the next processing cycle is a 3rd cycle (the Step S50: 3rd cycle), the pa_ex x 1.1 is set as the p (Step S52), and the processing in the Step S43 to the Step S47 are performed to calculate third values of the temporary period temp_period and the temporary weighted period temp_period_weighted.

On the other hand, when the flg_low_noise = 0 (the Step S48: NO), or when the flg_low_noise = 1 and the processing of calculating the temporary period temp_period have been performed three times (the Step S49: YES), it is determined whether or not the temporary period temp_period has been calculated, by referring the flag flg_same (Step S53).

When at least one of the calculation flags flg_same for the three values of the p (the Step S53: YES) is 1, an average of the temporary periods temp_period in the case where the calculation flags flg_same in the three processing cycles for the three values of the p (the temporary period temp_period in the case where the calculation flag flg_same in one processing cycle for one of the three values of the p when flg_low_noise = 0) is calculated as a temporal average period temp_ave_period (Step S54).

On the other hand, when no temporary period temp_period has been calculated (Step S53: NO), among three temporary weighted periods temp_period_weighted, one temporary weighted period corresponding to the greatest frequency max_freq_weighted (one temporary weighted periods temp_period_weighted when flg_low_noise = 0) is used as the temporal average period temp_ave_period (Step S55). In the Step S54, the temporal average period temp_ave-period may be derived by subjecting the temporary periods temp_period to averaging with weighting according to respective frequencies corresponding to the temporary periods temp_period.

Subsequently, when there is a large difference between the temporal average period temp_ave_period and an average period ave_period derived in the last measurement (Step S56: YES), adjustment of the temporal average period temp_ave_period is performed (Step S57), whereas, when there is a small difference therebetween (the Step S56: NO), the adjustment is not performed. Specifically, when the temporal average period temp_ave_period in this measurement is less than 70% of the average period ave_period in the last measurement, a value obtained by multiplying the average period ave_period in the last measurement by 0.7 is used as a temporal average period temp_ave_period in this measurement. On the other hand, when the temporal average period temp_ave_period in this measurement is 1.3 times greater than the average period ave_period in the last measurement, a value obtained by multiplying the average period ave_period in the last measurement by 1.3 is used as a temporal average period temp_ave_period in this measurement.

Subsequently, an average of the temporal average period temp_ave_period in this measurement and a predetermined number of previous temporal average periods temp_ave_period is calculated as an average period ave_period in this measurement (Step S58).

From this average period ave_period, a pulse rate ave_PR is calculated (Step S59) (ave_PR = 60 / ave_period)

< Method of determining effective greatest frequency zone and effective greatest weighted-frequency zone >

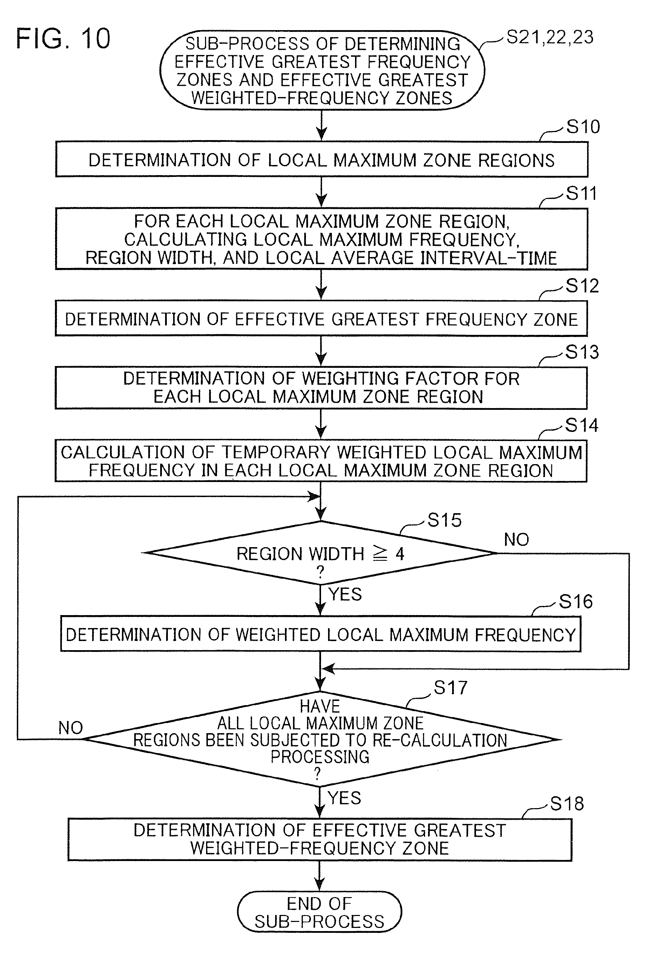

With reference to FIG. 10, a method of determining an effective greatest frequency zone and an effective greatest weighted-frequency zone from a frequency distribution will be described below. FIG. 10 is a flowchart illustrating a processing of determining an effective greatest frequency zone and an effective greatest weighted-frequency zone. This flowchart is common to the processing (Step S21, Step S22 and Step S23) for three types of intervals, i.e., the peak, valley and rising intervals. Therefore, only the processing for peak intervals will be representatively described here.

First of all, a plurality of local maximum zone regions are determined (Step S10). Specifically, a plurality of local maximum zones each of which is a class interval providing a local maximum in a frequency distribution (see extreme values 50 to 53 in FIG. 4) are derived, and a valley-to-valley region including each of the local maximum zones in the frequency distribution is determined as a local maximum zone region. In FIG. 4, the local maximum zone region is described as "local maximum region". It is highly likely that a zone of the first local maximum value 50 or a zone of the last local maximum value 53 does not include a true pulse-wave period. Thus, regarding the zone of the first local maximum value 50 or the last local maximum value 53, only when the first local maximum value 50 or the last local maximum value 53 is greater than, e.g., 1.5 times or more greater than, the remaining local maximum values, the zone is determined as a local maximum zone.

Subsequently, for each of the local maximum zone regions, a local maximum frequency and a region width (the number of class intervals making up the local maximum zone region) are derived. Further, an average interval-time of peak intervals included in the local maximum zone region is derived (Step S11).

Subsequently, an effective greatest frequency zone is determined (Step S12). In the local maximum zone regions, a greatest one of the local maximum frequencies derived in the Step S11 is determined as an effective greatest frequency, and a class interval corresponding to the effective greatest frequency is determined as an effective greatest frequency zone. In FIG. 4, the local maximum zone region having the greatest local maximum frequency is described as "effective greatest local maximum region".

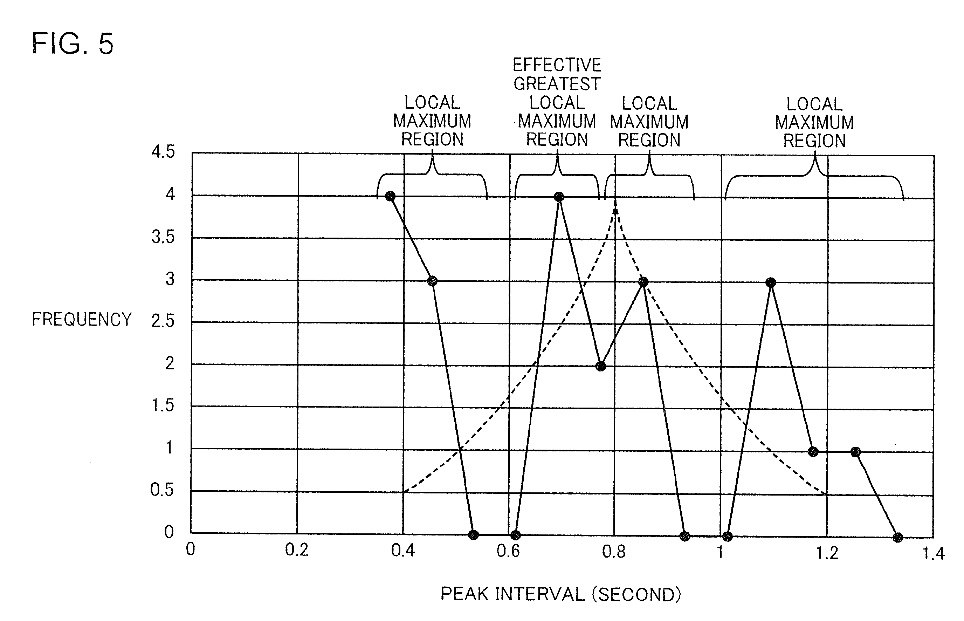

Subsequently, for each of the local maximum zone regions, a weighting factor is determined based on the average interval-time derived in the Step S11 and a period (ave_period) calculated in the last measurement (Step S13). For example, in the case where the ave_period is 0.8 sec, the weighting factor is set to a highest value for a class interval including 0.8 sec, and reduced with an increase in deviation from 0.8 sec, as indicated by the dashed line in FIG. 5.

Subsequently, a temporary weighted local maximum frequency in each of the local maximum zone regions is calculated (Step S 14). Specifically, for each of the local maximum zone regions, a frequency in each class interval of the local maximum zone region is multiplied by a weighting factor for each class interval, to thereby derive a temporary weighted local maximum frequency in the local maximum zone region.

Subsequently, for each of the local maximum zone regions (Step S 17: NO), the temporary weighted local maximum frequency is subjected to re-calculation depending on the region width derived in the Step S11. Specifically, when the region width is less than a predetermined value, e.g., "4" (Step S15: NO), the temporary weighted local maximum frequency derived in the Step S14 is directly determined as a weighted local maximum without re-calculation, whereas, when the region width is equal to or greater than the predetermined value (the Step S15: YES), the temporary weighted local maximum frequency derived in the Step S14 is subjected to re-calculation using the following formula (11) to derive a weighted local maximum frequency.

$$\text{Weighted local maximum frequency} = \text{temporary weighted local maximum frequency} \times 3 \ / \ \text{region width}$$

Upon completion of the processing for re-calculation of weighted local maximum frequency in all of the local maximum zone regions (Step S17: YES), an effective greatest weighted-frequency zone is determined (Step S18). Specifically, in the local maximum zone regions, a greatest one of the weighted local maximum frequencies derived in Steps S 14 to S 17 is determined as an effective greatest weighted-frequency, and the local maximum zone region corresponding to the effective weighted local maximum frequency is determined as effective greatest weighted-frequency zone.

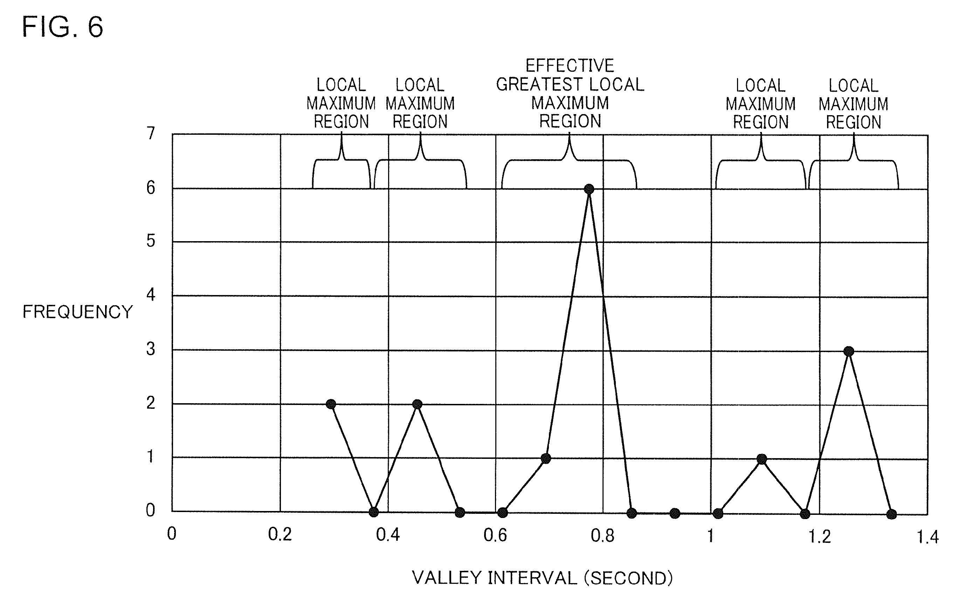

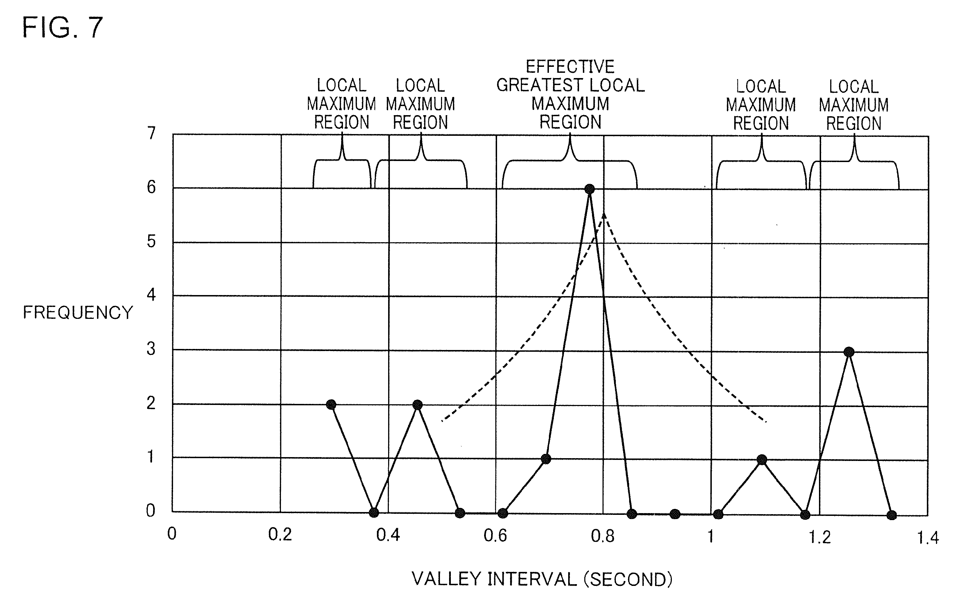

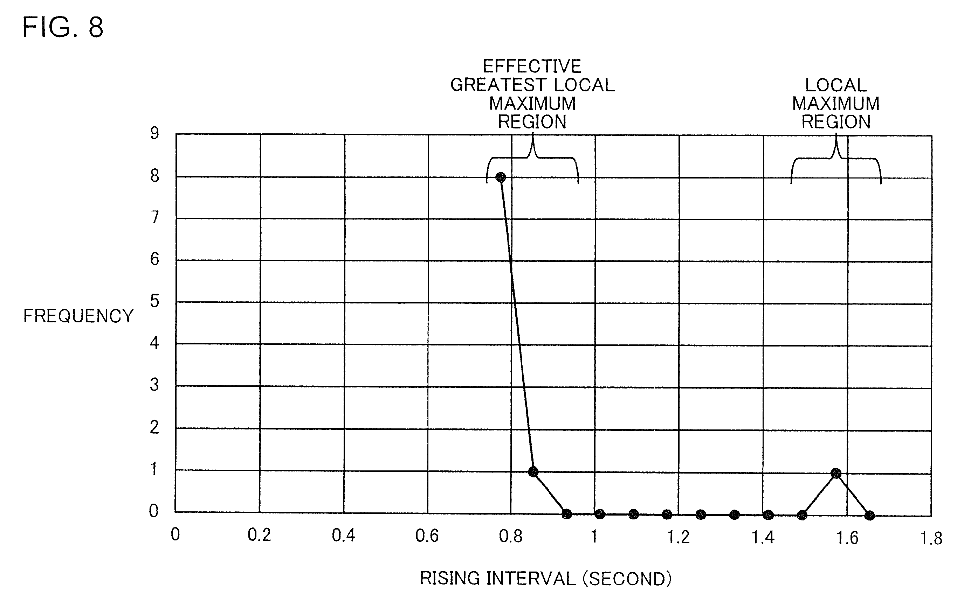

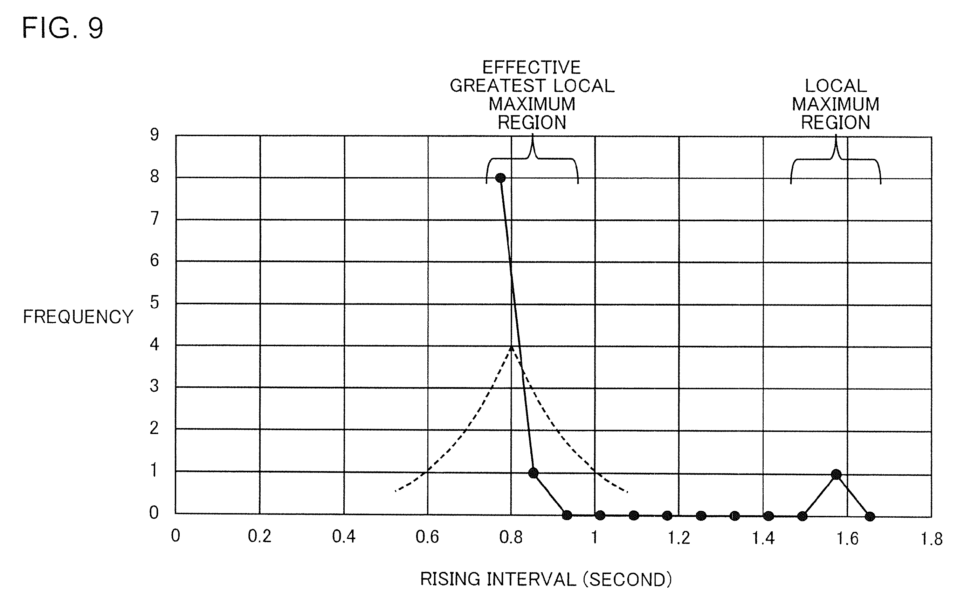

FIG. 6 illustrates one example of a valley-interval frequency distribution, and FIG. 7 illustrates one example of a weighting factor for valley. Further, FIG. 8 illustrates one example of a rising-interval frequency distribution, and FIG. 9 illustrates one example of a weighting factor for rising.

< Configuration >

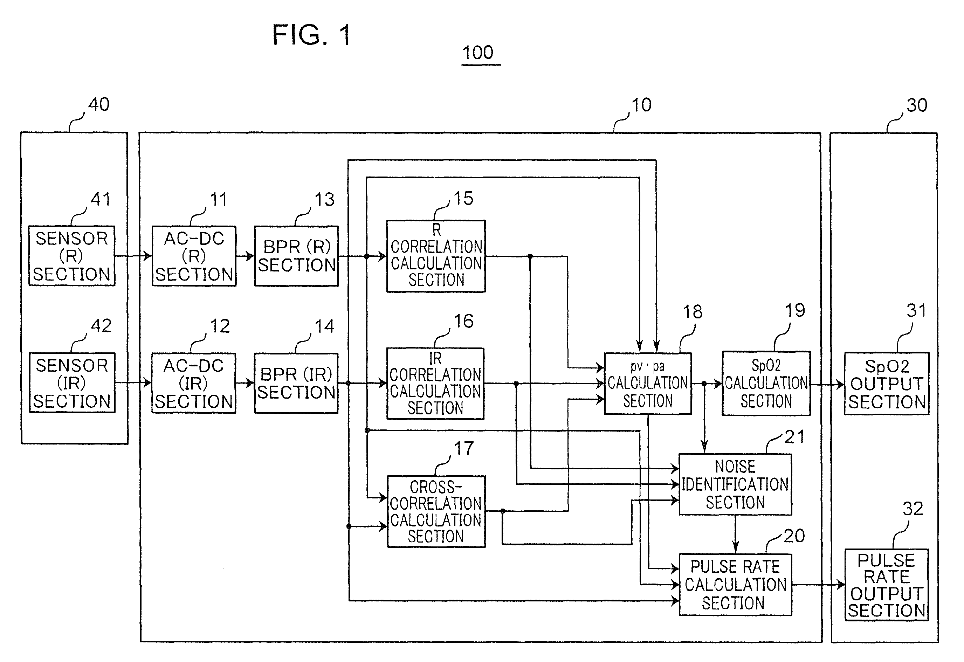

A biological information measuring apparatus according to one embodiment will be described below. FIG. 1 is a block diagram illustrating a configuration of the biological information measuring apparatus according to this embodiment.

The biological information measuring apparatus 100 according to this embodiment is an apparatus configured to measure a physiological phenomenon of a living body as a measurement target to thereby measure biological information regarding the living body. Examples of the biological information include pulse rate and blood oxygen saturation. Such biological information can be derived by utilizing a fluctuation component occurring in an intensity of light transmitted through or reflected by a biological tissue, due to pulsation of arterial blood, and a basic principle thereof is as described in the aforementioned Section < Principle of present invention >.

For example, as illustrated in FIG. 1, the biological information measuring apparatus 100 comprises: a sensor unit 40 for measuring a given physiological phenomenon of a living body and outputting measurement data; an operational control unit 10 for calculating biological information such as pulse rate and blood oxygen saturation (SpO2, $SpO_2$,

percutaneous oxygen saturation), based on measurement data measured by the sensor unit 40; and an indicating unit 30 for indicating biological information calculated by the operational control unit 10 in an externally recognizable manner.

The sensor unit 40 is connected to the operational control unit 10. In this embodiment, it is a device configured to measure information about blood in a biological tissue, as a physiological phenomenon of a living body. More specifically, the sensor unit 40 is a device configured to measure a fluctuation component occurring in an intensity of light transmitted through or reflected by a biological tissue, due to pulsation of arterial blood according to heartbeat.

Examples of a method of measuring such a physiological phenomenon include a method utilizing light-absorption properties of hemoglobin in a biological tissue. Oxygen is carried to biological cells by hemoglobin, wherein hemoglobin is combined with oxygen to form oxygenated hemoglobin, and oxygenated hemoglobin returns to hemoglobin (reduced hemoglobin) when oxygen is consumed in biological cells. The blood oxygen saturation is defined as a rate of oxygenated hemoglobin in blood. An absorbance of each of hemoglobin and oxygenated hemoglobin has a wavelength dependence. For example, in regard to red light (light in a red wavelength region), hemoglobin absorbs more light than oxygenated hemoglobin, and, in regard to infrared light (light in an infrared wavelength region), it absorbs less light than oxygenated hemoglobin. That is, hemoglobin has an optical property that, when it is oxidized into oxygenated hemoglobin, its red light absorbability is deteriorated and an infrared absorbability is enhanced, whereas, when oxygenated hemoglobin is reduced to return to hemoglobin, the red light absorbability is enhanced and the infrared is deteriorated. The biological information measuring apparatus 100 according to this embodiment is designed to derive biological information such as pulse rate and blood oxygen saturation by utilizing a difference in light absorption properties between hemoglobin and oxygenated hemoglobin, in regard to red light and infrared light.

Because of such a methodology, for example, the sensor unit 40 in this embodiment comprises a sensor (R) section 41 for measuring a light absorption property of a biological tissue in regard to red light, and a sensor (IR) section 42 for measuring a light absorption property of the biological tissue in regard to infrared light, wherein each of them is connected to the operational control unit 10. For example, the sensor (R) section 41 comprises an R light-emitting element, such as a light-emitting diode, configured to emit red light having a wavelength $\lambda 1$ to the biological tissue, and an R light-receiving element, such as a silicon photodiode, configured to receive red light transmitted through or reflected by the biological tissue after being emitted from the R light-emitting element, and the sensor (IR) section 42 comprises an IR light-emitting element, such as a light-emitting diode, configured to emit infrared light having a wavelength $\lambda 2$ different from the wavelength $\lambda 1$ to the biological tissue, and an IR light-receiving element, such as a silicon photodiode, configured to receive infrared light transmitted through or reflected by the biological tissue after being emitted from the IR light-emitting element. The sensor unit 40 may employ a transmission-type or reflection-type sensor having the above functions.

The sensor unit 40 is configured to output measurement data measured by the sensor (R) section 41 and the sensor (IR) section 42 to the operational control unit 10, while being set on a given biological tissue of a finger or an earlobe, or, in case of an infant, the back of a hand, a wrist, the top of a foot or the like, and, in this state. More specifically, in the sensor (R) section 41 having the above configuration, the R light-emitting element is operable to emit red light to the biological tissue, and the R light-receiving element is operable to receive red light R transmitted through or reflected by the biological tissue after being emitted from the R light-emitting element to the biological tissue, and subject the received red light to photoelectric conversion to thereby output an electric signal depending on an intensity of the received red light to the operational control unit 10, as the measurement data. Similarly, in the sensor (IR) section 42, the IR light-emitting element is operable to emit infrared light to the biological tissue, and the IR light-receiving element is operable to receive infrared light transmitted through or reflected by the biological tissue after being emitted from the IR light-emitting element to the biological tissue, and subject the received infrared light to photoelectric conversion to thereby output an electric signal depending on an intensity of the received infrared light to the operational control unit 10, as the measurement data. In this embodiment, two light-receiving elements, i.e., the R light-receiving element and the IR light-receiving element, are provided. Alternatively, a single light-receiving element may be provided. In this case, the R light-emitting element and the IR light-emitting element may be configured to emit light at respective timings shifted from each other (emit light in a time-sharing manner), to allow an output of the single light-receiving element to be separated into an electric signal associated with the IR light-emitting element and an electric signal associated with the R light-emitting element, according to respective times corresponding to the light-emitting timings.

The operational control unit 10 is a device connected to the indicating unit 30 and configured to derive biological information based on measurement data measured by the sensor unit 40 and govern control of the entire biological information measuring apparatus 100. For example, the operational control unit 10 is configured to sample measurement data measured by the sensor unit 40 with a given sampling period (e.g., frequency: 37.5 Hz) to thereby acquire a time-series data about measurement data, from the sensor unit 40. Further, for example, the operational control unit 10 is configured to drive the sensor unit 40 with a given period, i.e., instruct the sensor unit 40 to perform light-emitting and light-receiving operations, to thereby acquire measurement data from the sensor unit 40 in the form of a time-series data. Further, for example, the sensor unit 40 is configured to perform sampling with a given sampling period to measure measurement data from the biological tissue in the form of a time-series data, and output the time-series data about the measurement data to the operational control unit 10. In this embodiment, this measurement data is digital data although it may be analog data. Conversion from analog data to digital data (A-D conversion) may be performed by the sensor unit 40 or the operational control unit 10. Further, according to need, an amplification section for amplifying measurement data before the A-D conversion may be additionally provided in the sensor unit 40 or the operational control unit 10.

More specifically, the operational control unit 10 is designed to derive given biological information regarding a living body as a measurement target, based on measurement data measured by the sensor unit 40, and composed, for example, of a microcomputer comprising a microprocessor, a memory and a peripheral circuit thereof. The memory comprises: a storage element, such as an EEPROM (Electrically Erasable Programmable Read Only Memory) as a re-writable, non-volatile storage element or a ROM (Read Only Memory) as a non-volatile storage element, which stores therein various programs such as a biological information operation program for deriving biological information based on measurement data measured by the sensor unit 40 and a control program for controlling the entire biological information measuring apparatus 100, and various data such as the measurement data measured by the sensor unit 40 and data necessary for executing the programs; and a storage element serving as a so-called working memory for the microprocessor, such as a RAM (Random Access Memory) as a volatile storage element. For example, the microprocessor consists of a so-called CPU (Central Processing Unit) or the like, and, functionally, during execution of the programs, comprises: an AC-DC (R) section 11; an AC-DC (IR) section 12; a BPR (R) section 13; a BPR (IR) section 14; an R correlation calculation section 15; an IR correlation calculation section 16; a cross-correlation calculation section 17; a pv · pa calculation section 18; SpO2 calculation section 19; a pulse rate calculation section 20; and a noise discrimination section 21.

Each of the AC-DC (R) section 11 and the AC-DC (IR) section 12 is designed to subject measurement data input from the sensor unit 40 to a give pre-processing. More specifically, the AC-DC (R) section 11 is configured to subject red light-related measurement data input from the sensor (R) section 41 to a so-called dark processing for correcting a dark current in the R light-receiving element, and calculate a first ratio (red light AC/DC ratio) R of an AC component RAC to a DC component RDC (= RAC/RDC) to notify the BPR (R) section 13 of the first ratio R (output the first ratio R to the BPR (R) section 13).

The AC-DC (IR) section 12 is configured to subject infrared light-related measurement data input from the sensor (IR) section 42 to a so-called dark processing for correcting a dark current in the IR light-receiving element, and calculate a second ratio (infrared light AC/DC ratio) IR of an AC component IRAC to a DC component IRDC (= IRAC/IRDC) to notify the BPR (IR) section 14 of the second ratio IR (output the second ratio IR to the BPR (IR) section 14). The dark processing is performed by using a heretofore-known method, for example, by subtracting an output value (dark current value) Rdark which is output from the R light-receiving element in a light-shielded state, from the red light-related measurement data, and subtracting an output value (dark current value) IRdark which is output from the IR light-receiving element in a light-shielded state, from the infrared light-related measurement data. The output values Rdark, IRdark which are output from the respective R and IR light-receiving elements in the light-shielded state are preliminarily measured.

Each of the BPR (R) section 13 and the BPR (IR) section 14 is a filter for removing a given noise component from measurement date measured by the sensor unit 40, and designed to remove any frequency component other than a frequency component normally included as a fluctuation component occurring in an intensity of light transmitted through or reflected by a biological tissue, due to pulsation of arterial blood.

The BPR (R) section 13 is a filter configured for red light to have a passband set to a given frequency band including a frequency component normally included as a fluctuation component occurring in an intensity of light transmitted through or reflected by a biological tissue, due to pulsation of arterial blood, and configured to subject the first ratio R to a filter processing (filtering) and notify the R correlation calculation section 15, the cross-correlation calculation section 17, the pv · pa calculation section 18, the pulse rate calculation section 20 and the noise discrimination section 21 of the filtered first ratio R in the form of time-series data R_signal.

The BPR (IR) section 14 is a filter configured for infrared light to have a passband set to a given frequency band including a frequency component normally included as a fluctuation component occurring in an intensity of light transmitted through or reflected by a biological tissue, due to pulsation of arterial blood, and configured to subject the second ratio IR to a filter processing (filtering) and notify the IR correlation calculation section 16, the cross-correlation calculation section 17, the pv · pa calculation section 18, the pulse rate calculation section 20 and the noise discrimination section 21 of the filtered second ratio R in the form of time-series data IR_signal.

The R correlation calculation section 15 is configured to calculate correlation data about the red light-related R_signal or a difference signal $\Delta R$ of the R_signal, and notify the pv · pa calculation section 18 and the noise discrimination section 21 of the calculated correlation data.

The IR correlation calculation section 16 is configured to calculate correlation data about the infrared light-related IR_signal or a difference signal $\Delta IR$ of the IR_signal, and notify the pv calculation section 18 and the noise discrimination section 21 of the calculated correlation data.

The cross-correlation calculation section 17 is configured to calculate correlation data between the red light-related R_signal or the difference signal $\Delta R$ thereof and the infrared light-related IR_signal or the difference signal $\Delta IR$ thereof, and notify the pv · pa calculation section 18 and the noise discrimination section 21 of the calculated correlation data.

The pv · pa calculation section 18 is configured to calculate an estimate kv_es of the k_v from the R_signal calculated by the BPR (R) section 13, the IR_signal calculated by the BPR (IR) section 14, the correlation signal about the R_signal or the difference signal $\Delta R$ thereof calculated by the R correlation calculation section 15, the correlation signal about the IR_signal or the difference signal $\Delta IR$ thereof calculated by the IR correlation calculation section 16, and the correlation data between the R_signal or the difference signal $\Delta R$ thereof and the IR_signal or the difference signal $\Delta IR$ thereof calculated by the cross-correlation calculation section 17, and by using a conventional technique. For example, the pv · pa calculation section 18 is operable to calculate a variation in width between waveforms (IR_signal $\times$ k_v - R_signal) obtained by sequentially changing the k_v from an initial value k_v_0 within a given range (e.g., k_v_0 - 0.5 < k_v < k_v_0 + 0.5), and determine the k_v in such a manner as minimize the variation, to thereby derive the kv_es.

The pv · pa calculation section 18 is operable, based on the derived value of the kv_es, to calculate the estimate pv_es for venous blood, and then calculate the estimate pa_es of the k_a by using the formula (8). Then, the pv · pa calculation section 18 is operable to notify the SpO2 calculation section 19, the pulse rate calculation section 20 and the noise discrimination section 21 of the calculated values of the pv_es and pa_es.

The SpO2 calculation section 19 is configured to derive the arterial oxygen saturation SpO2 from the pa_es derived by the pv · pa calculation section 18, and notify the indicating unit 30 of the derived value of the arterial oxygen saturation SpO2.

The pulse rate calculation section 20 is configured to derive the pulse rate ave_PR from the R_signal calculated by the BPR (R) section 13, the IR_signal calculated by the BPR (IR) section 14 and the pv_es and pa_es calculated by the pv · pa calculation section 18, as described in connection with FIG. 13, and notify the indicating unit 30 of the calculated value of the pulse rate ave_PR.

The noise discrimination section 21 is configured to calculate the noise index from the correlation signal about the R_signal or the difference signal $\Delta R$ thereof calculated by the R correlation calculation section 15, the correlation signal about the IR_signal or the difference signal $\Delta IR$ thereof calculated by the IR correlation calculation section 16, the correlation data between the R_signal or the difference signal $\Delta R$ thereof and the IR_signal or the difference signal $\Delta IR$ thereof calculated by the cross-correlation calculation section 17, and the pv_es and pa_es calculated by the pv · pa calculation section 18, and by using the formula (10). Further, it is configured to determine that the arterial blood noise is large, when the calculated value of the noise index is less than a predetermined threshold, or determine that that the noise is small, when the calculated value of the noise index is equal to or greater than the predetermined threshold, and notify the pulse rate calculation section 20 of a result of the determination.

The indicating unit 30 is a device configured to indicate (output) a state of operation of the biological information measuring apparatus 100, biological information derived by the operational control unit 10, and composed, for example, of a liquid crystal display (LCD) unit, an organic EL display unit, a printer, or the like. For example, in this embodiment, the indicating unit 30 comprises a pulse rate output section 32 for indicating a value of pulse rate calculated by the pulse rate calculation section 20 and a value of oxygen saturation calculated by the SpO2 calculation section 19.

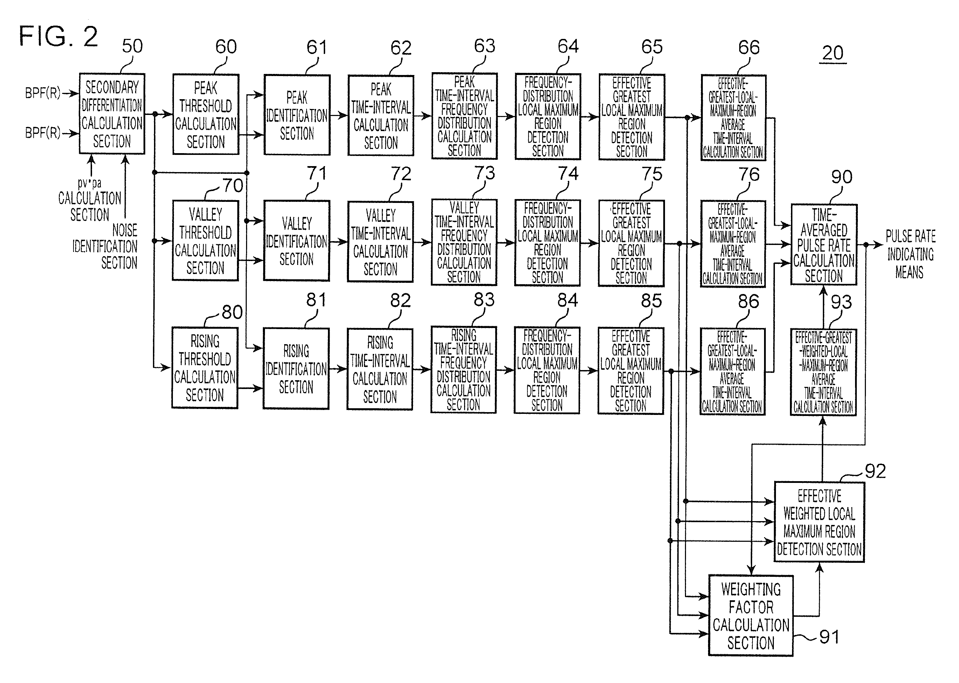

FIG. 2 is a block diagram illustrating a configuration of the pulse rate calculation section 20 in the biological information processing apparatus 100 in FIG. 1.

The pulse rate calculation section 20 comprises a secondary differentiation calculation section 50, a peak threshold calculation section 60, a peak discrimination section 61, a peak time-interval calculation section 62, a peak time-interval frequency distribution calculation section 63, a frequency-distribution local maximum region detection section 64, an effective greatest local maximum region detection section 65, an effective-greatest-local-maximum-region average time-interval calculation section 66, a valley threshold calculation section 70, a valley discrimination section 71, a valley time-interval calculation section 72, a valley time-interval frequency distribution calculation section 73, a frequency-distribution local maximum region detection section 74, an effective greatest local maximum region detection section 75, an effective-greatest-local-maximum-region average time-interval calculation section 76, a rising threshold calculation section 80, a rising discrimination section 81, a rising time-interval calculation section 82, a rising time-interval frequency distribution calculation section 83, a frequency-distribution local maximum region detection section 84, an effective greatest local maximum region detection section 85, an effective-greatest-local-maximum-region average time-interval calculation section 86, a time-averaged pulse rate calculation section 90, a weighting factor calculation section 91, an effective weighted local maximum region detection section 92. and an effective-greatest-weighted-local-maximum-region average time-interval calculation section 93.

The secondary differentiation calculation section 50 is configured to perform a processing of generating a second-order difference signal R_signal - p $\times$ IR_signal. This processing corresponds to the processing in the Step S44 of the flowchart in FIG. 13.

Each of the peak threshold calculation section 60, the valley threshold calculation section 70 and the rising threshold calculation section 80 is configured to perform a processing of calculating a threshold for detecting a point of a respective one of peak, valley and rising in the second-order difference signal generated by the secondary differentiation calculation section 50. This processing corresponds to the processing in the Step S45 of the flowchart in FIG. 13.

Each of the peak discrimination section 61, the valley discrimination section 71 and the rising discrimination section 81 is configured to perform a processing of detecting a point of a respective one of peak, valley and rising in the second-order difference signal generated by the secondary differentiation calculation section 50, based on a respective one of the thresholds calculated by the peak threshold calculation section 60, the valley threshold calculation section 70 and the rising threshold calculation section 80. This processing corresponds to the processing in the Step S46 of the flowchart in FIG. 13.

The peak time-interval calculation section 62 is configured to calculate an interval-time between peaks detected by the peak discrimination section 61, and the peak time-interval frequency distribution calculation section 63 is configured to perform a processing of creating a frequency distribution from the calculated interval-times. This processing corresponds to the processing in the Step S20 of the flowchart in FIG. 11.

The frequency-distribution local maximum region detection section 64 is configured to perform a processing of detecting a local maximum zone region from the frequency distribution created by the peak time-interval frequency distribution calculation section 63. This processing corresponds to the processing in the Step S10 of the flowchart in FIG. 10.

The effective greatest local maximum region detection section 65 is configured to perform a processing of determining an effective greatest local maximum region (effective greatest frequency zone) from the local maximum zone region in the peak time-interval frequency distribution, detected by the frequency-distribution local maximum region detection section 64. This processing corresponds to the processing in the Steps S11 and S12 of the flowchart in FIG. 10.

The effective-greatest-local-maximum-region average time-interval calculation section 66 is configured to perform a processing of calculating an average time (period) from the interval-times in the effective greatest local maximum region determined by the effective greatest local maximum region detection section 65. This processing corresponds to the processing in the Step S24 of the flowchart in FIG. 11.

The valley time-interval calculation section 72 is configured to calculate an interval-time between valleys detected by the valley discrimination section 71, and the valley time-interval frequency distribution calculation section 73 is configured to perform a processing of creating a frequency distribution from the calculated interval-times. This processing corresponds to the processing in the Step S20 of the flowchart in FIG. 11.

The frequency-distribution local maximum region detection section 74 is configured to perform a processing of detecting a local maximum zone region from the frequency distribution created by the peak time-interval frequency distribution calculation section 73. This processing corresponds to the processing in the Step S10 of the flowchart in FIG. 10.

The effective greatest local maximum region detection section 75 is configured to perform a processing of determining an effective greatest local maximum region (effective greatest frequency zone) from the local maximum zone region in the valley time-interval frequency distribution, detected by the frequency-distribution local maximum region detection section 74. This processing corresponds to the processing in the Steps S11 and S12 of the flowchart in FIG. 10.

The effective-greatest-local-maximum-region average time-interval calculation section 76 is configured to perform a processing of calculating an average time (period) from the interval-times in the effective greatest local maximum region determined by the effective greatest local maximum region detection section 75. This processing corresponds to the processing in the Step S25 of the flowchart in FIG. 11.

The rising time-interval calculation section 82 is configured to calculate an interval-time between risings detected by the rising discrimination section 81, and the rising time-interval frequency distribution calculation section 83 is configured to perform a processing of creating a frequency distribution from the calculated interval-times. This processing corresponds to the processing in the Step S20 of the flowchart in FIG. 11.

The frequency-distribution local maximum region detection section 84 is configured to perform a processing of detecting a local maximum zone region from the frequency distribution created by the rising time-interval frequency distribution calculation section 83. This processing corresponds to the processing in the Step S10 of the flowchart in FIG. 10.

The effective greatest local maximum region detection section 85 is configured to perform a processing of determining an effective greatest local maximum region (effective greatest frequency zone) from the local maximum zone region in the rising time-interval frequency distribution, detected by the frequency-distribution local maximum region detection section 84. This processing corresponds to the processing in the Steps S11 and S12 of the flowchart in FIG. 10.

The effective-greatest-local-maximum-region average time-interval calculation section 86 is configured to perform a processing of calculating an average time (period) from the interval-times in the effective greatest local maximum region determined by the effective greatest local maximum region detection section 75. This processing corresponds to the processing in the Step S26 of the flowchart in FIG. 11.

The time-averaged pulse rate calculation section 90 is configured to perform a processing of calculating pulse rate from the average times (periods) calculated by the effective-greatest-local-maximum-region average time-interval calculation section 66, the effective-greatest-local-maximum-region average time-interval calculation section 76, and the effective-greatest-local-maximum-region average time-interval calculation section 86. This processing corresponds to the processing in the Step S27 to the Step 33 of the flowchart in FIG. 12, and the Step S48 to the Step 59 of the flowchart in FIG. 13.

The weighting factor calculation section 91 is configured to perform a processing of calculating a weighting factor. This processing corresponds to the processing in the Step S 13 of the flowchart in FIG. 10.

The effective weighted local maximum region detection section 92 is configured to perform a processing of detecting an effective greatest weighted local maximum region (effective greatest weighted-frequency zone) by using the weighting factor calculated by the weighting factor calculation section 91. This processing corresponds to the processing in the Steps S 14 to 18 of the flowchart in FIG. 10.

The effective-greatest-weighted-local-maximum-region average time-interval calculation section 93 is configured to perform a processing of calculating an average time (period) from the interval-times in the effective greatest weighted local maximum region detected by the effective weighted local maximum region detection section 92. This processing corresponds to the processing in the Step S34 of the flowchart in FIG. 12.

< Operation >

Next, an operation of the biological information measuring apparatus 100 will be described.

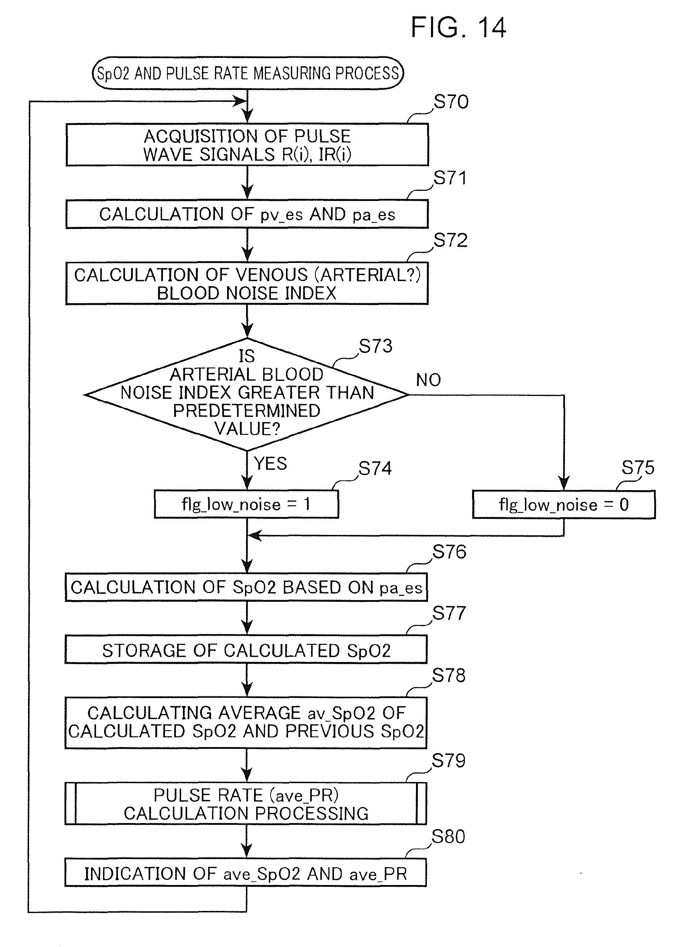

FIG. 14 is a flowchart illustrating a process of deriving oxygen saturation and pulse rate in biological information.

In the biological information measuring apparatus 100, a measurement of biological information of a living body as a measurement target is started upon turn-on of a power switch (not illustrated) or turn-on of a measurement start switch (not illustrated) after turn-on of the power switch.

The operational control unit 10 starts to sample measurement data measured by the sensor unit 40, with a given sampling period.

Specifically, in the sensor unit 41, red light-related measurement data Rsignalanddark (including dark current) and a dark current Rdark therein are measured by the sensor (R) section 41, and converted from an analog signal to a digital signal, and infrared light-related measurement data IRsignalanddark (including dark current) and a dark current IRdark therein are measured by the sensor (IR) section 42 and converted from an analog signal to a digital signal.

Subsequently, in the indicating unit 30, the AC-DC (R) section 11 operates to subject the red light-related measurement data Rsignalanddark input from the sensor (R) section 41 to a dark processing (Rsignalanddark - Rdark) to thereby derive R_signal, and the AC-DC (R) section 11 operates to subject the infrared light-related measurement data IRsignalanddark input from the sensor (IR) section 42 to a dark processing (Rsignalanddark - Rdark) to thereby derive IR_signal. Subsequently, the R_signal output from the AC-DC (R) section 11 and the I R_signal output from the AC-DC (IR) section 12 are filtered, respectively, by the BPR (R) section 13 and the BPR (IR) section 14, and then output to the R correlation calculation section 15 and others.

The operational control unit 10 starts data sampling. Each of the R correlation calculation section 15, the IR correlation calculation section 16 and the cross-correlation calculation section 17 acquires the R_signal (in FIG. 14, described as R(i)) or IR_signal (in FIG. 14, described as IR(i)) from the BPR (R) section 13 or the BPR (IR) section 14 (Step S70) to calculate correlation values, and output the correlation values to the pv · pa calculation section 18. That is, $\sum R(i)^2$ or $\sum \Delta R(i)^2$, $\sum IR(i)^2$ or $\sum \Delta IR(i)^2$, and $\sum R(i) \times IR(i)$ or $\sum \Delta R(i) \times \sum \Delta IR(i)$, are calculated, respectively, by the R correlation calculation section 15, the IR correlation calculation section 16 and the IR correlation calculation section 16, and then output to the pv · pa calculation section 18. $\Delta R(i)$ and $\Delta IR(i)$ represent, respectively, temporal differences in R(i) and IR(i), and $\sum$ represents a sum within a given period of time.

Upon acquiring a given number of (N) data necessary for calculating oxygen saturation and pulse rate, the pv · pa calculation section 18 calculates the pv_es and pa_es, and outputs calculated values of the pv_es and pa_es to the SpO2 calculation section 19, the pulse rate calculation section 20 and the noise discrimination section 21 (Step S71).

The noise discrimination section 21 calculates the noise index by using the formula (10) (Step S72). When the calculated noise index is less than a predetermined threshold (Step S73: YES), it determines that arterial blood noise is large, and sets flg_low_noise to 1 (Step S74). On the other hand, when the calculated noise index is equal to or greater than the threshold (the Step S73: NO), the noise discrimination section 21 determines that arterial blood noise is small, and sets flg_low_noise to 0 (Step S75). Then, it sends the content of the flag to the pulse rate calculation section 20.

The SpO2 calculation section 23 derives the oxygen saturation (SpO2) from the estimate pa_es calculated by the pv · pa calculation section 18 (Step S76), and stores the derived value of the oxygen saturation SpO2 in the memory of the biological information measuring apparatus 100 (Step S77). Then, in the case where previously-calculated values of oxygen saturation SpO2 are stored, the SpO2 calculation section 23 calculates an average of values of oxygen saturation SpO2 previously calculated over a given period of time, and the currently-calculated value of the oxygen saturation SpO2, and notifies the indicating section 30 of the calculated oxygen saturation SpO2 ave_SpO2 (Step S78).

The pulse rate calculation section 20 calculates pulse rate from the content of the flag flg_low_noise notified from the noise discrimination section 21, the pv_es and pa_es received from the pv · pa calculation section 18, the R(i) received from the BPR (R) section 13, and IR(i) received from the BPR (IR) section 14, as described in connection with FIG. 13, and notifies the indicating section 30 of the calculated value of the pulse rate ave_PR (Step S79).

The indicating section 30 indicates the value of the oxygen saturation SpO2 ave_SpO2 notified from the SpO2 calculation section 19 a, and the value of the pulse rate ave_PR notified from the pulse rate calculation section 20, respectively, on the SpO2 output section 31 and the pulse rate output section 32 (Step S80).

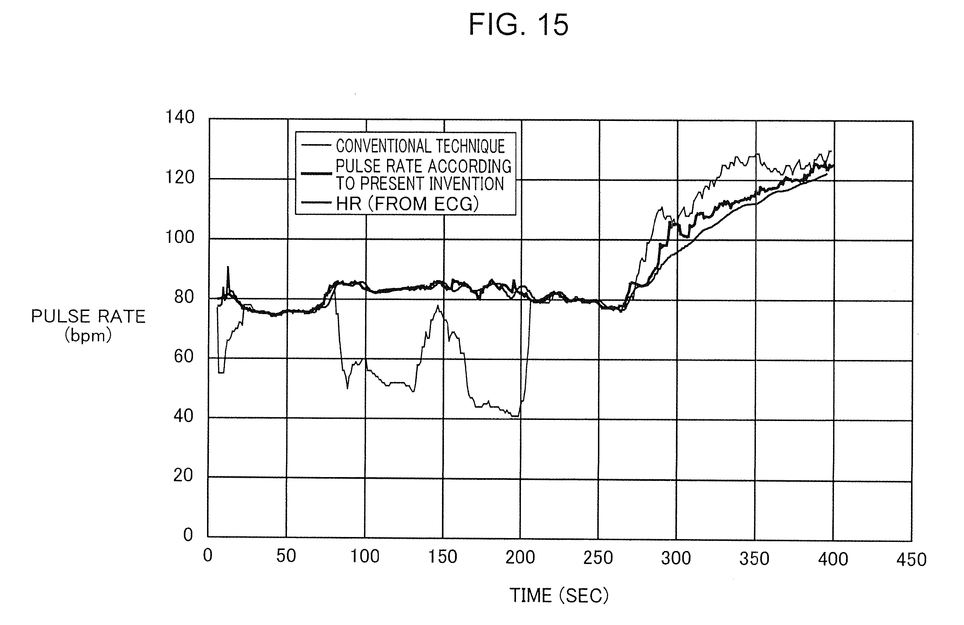

The biological information measuring apparatus 100 according to this embodiment operates in the above manner. Thus, even when noise occurs due to walking or the like, the biological information measuring apparatus 100 can measure biological information such as pulse rate and oxygen saturation, accurately and quickly. FIG. 15 illustrates one example of a pulse rate curve during walking, measured by the above biological information processing apparatus. As presented in this graph, it is proven that pulse rate measured by the biological information processing apparatus according to this embodiment runs on in approximately the same pattern as pulse rate of an electrocardiogram (HR graph), and calculates pulse rate more accurately than the conventional technique.

This specification discloses techniques having various aspects. Among them, major techniques will be outlined below.

A biological information processing apparatus according to one aspect is designed to, based on at least first measurement data and second measurement data obtained by emitting a plurality of light beams having respective different wavelengths to a living body and receiving corresponding light beams transmitted through or reflected by the living body, measure biological information of the living body, and equipped with a first measurement section, a second measurement section, a biological signal generation section, a difference signal generation section, a frequency distribution generation section, and a period calculation section. The first measurement section is configured to measure the first measurement data, wherein the first measurement data comprises a first signal component having periodicity, and a first noise component. The second measurement section is configured to measure the second measurement data, wherein the second measurement data comprising a second signal component having a given first relationship with the first signal component, and a second noise component having a given second relationship with the first noise component. The difference signal generation section is configured to generate a biological signal including the first signal component based on an estimate of a given third relationship, the first measurement data and the second measurement data. The difference signal generation section is configured to generate a second-order difference signal by subjecting the biological signal to a second-order differencing operation. The frequency distribution generation section is configured to generate at least one frequency distribution selected from the group consisting of: a peak frequency distribution which represents a frequency distribution of an interval-time between peaks at each of which the second-order difference signal has a value equal to or greater than a given peak threshold; a valley frequency distribution which represents a frequency distribution of an interval-time between valleys at each of which the second-order difference signal has a value equal to or less than a given valley threshold; a rising frequency distribution which represents a frequency distribution of an interval-time between rising points at each of which a value of the second-order difference signal comes across a given threshold representing an intermediary value between the peaks and the valleys in a rising direction; and a falling frequency distribution which represents a frequency distribution of an interval-time between falling points at each of which a value of the second-order difference signal comes across the given threshold representing the intermediary value between the peaks and the valleys in a falling direction. The period calculation section is configured to, with respect to the frequency distribution generated by the frequency distribution generation section, determine an effective greatest frequency zone which is a zone having a greatest frequency of the interval-time, based on a given criterion, and calculate a period of the first signal component based on an average time interval in the effective greatest frequency zone.

A signal processing method according to another aspect of the present invention is designed for use in a biological information processing apparatus configured to, based on at least first measurement data and second measurement data obtained by emitting a plurality of light beams having respective different wavelengths to a living body and receiving corresponding light beams transmitted through or reflected by the living body, measure biological information of the living body, wherein: the first measurement data comprises a first signal component having periodicity, and a first noise component; and the second measurement data comprises a second signal component having a given first relationship with the first signal component, and a second noise component having a given second relationship with the first noise component. The signal processing method comprises a biological signal generation step, a difference signal generation step, a frequency distribution generation step and a period calculation step. The biological signal generation step is configured to generate a biological signal including the first signal component, based on an estimate of a given third relationship, the first measurement data and the second measurement data. The difference signal generation step is configured to generate a second-order difference signal by subjecting the biological signal to a second-order differencing operation. The frequency distribution generation step is configured to generate at least one frequency distribution selected from the group consisting of: a peak frequency distribution which represents a frequency distribution of an interval-time between peaks at each of which the second-order difference signal has a value equal to or greater than a given peak threshold; a valley frequency distribution which represents a frequency distribution of an interval-time between valleys at each of which the second-order difference signal has a value equal to or less than a given valley threshold; a rising frequency distribution which represents a frequency distribution of an interval-time between rising points at each of which a value of the second-order difference signal comes across a given threshold representing an intermediary value between the peaks and the valleys in a rising direction; and a falling frequency distribution which represents a frequency distribution of an interval-time between falling points at each of which a value of the second-order difference signal comes across the given threshold representing the intermediary value between the peaks and the valleys in a falling direction. The period calculation step is configured to, with respect to the frequency distribution generated by the frequency distribution generation section, calculating a period of the first signal component based on the interval-time having a greatest frequency.

In the above biological information processing apparatus, the estimate of the third relationship is used to generate a biological signal while allowing the first signal component to be more largely included therein, so that it becomes possible to reduce signal noise. In addition, in the above biological information processing apparatus, a frequency distribution of an interval-time of peak or the like is created by using a second-order difference waveform of the biological signal with reduced noise, and the period is calculated based on an interval-time having a greatest frequency, so that it becomes possible to derive pulse rate with less error.

In one specific embodiment, the above biological information processing apparatus which further comprises: an estimation section configured to output respective estimates of an arterial blood absorption coefficient ratio associated with arterial blood and a venous blood absorption coefficient ratio associated with venous blood, each included in the first measurement data and the second measurement data, and a noise discrimination section configured to discriminate whether or not the second noise component largely includes a specific noise component due to blood having a value of oxygen saturation close to that of arterial blood. In this embodiment, the biological signal generation section is operable, when the noise discrimination section discriminates that the second noise component does not largely include the specific noise component, to determine the estimate of the given third relationship based on only the estimate of the venous blood absorption coefficient ratio, and, when the noise discrimination section discriminates that the second noise component largely includes the specific noise component, to determine, as the estimate of the given third relationship, a value between the respective estimates of the arterial blood absorption coefficient ratio and the venous blood absorption coefficient ratio.

In this biological information processing apparatus, it becomes possible to discriminate a level of noise due to blood having a value of oxygen saturation close to that of arterial blood, and thereby estimate a period by using an adequate value of the estimate of the third relationship according to a level and property of noise. That is, in this biological information processing apparatus, it becomes possible to derive pulse rate with less error.

In another specific embodiment, in the above biological information processing apparatus, the frequency distribution generation section is operable, when generating the peak frequency distribution in a given time range of the second-order difference signal, to select a plurality of peaks, and measure an interval-time between a certain one of the selected peaks and each of two or more of the remaining peaks included in a predetermined given range including the certain peak, with respect to each of the selected peaks, thereby generating the peak frequency distribution by using the measured interval-times. The frequency distribution generation section is also operable, when generating the valley frequency distribution in a given time range of the second-order difference signal, to select a plurality of valley, and measure an interval-time between a certain one of the selected valley and each of two or more of the remaining valleys included in a predetermined given range including the certain valley, with respect to each of the selected valleys, thereby generating the valley frequency distribution by using the measured interval-times. The frequency distribution generation section is operable, when generating the rising frequency distribution in a given time range of the second-order difference signal, to select a plurality of rising points, and measure an interval-time between a certain one of the selected rising points and each of two or more of the remaining rising points included in a predetermined given range including the certain rising points, with respect to each of the selected rising points, thereby generating the rising frequency distribution by using the measured interval-times. Further, the frequency distribution generation section is operable, when generating the falling frequency distribution in a given time range of the second-order difference signal, to select a plurality of falling points, and measure an interval-time between a certain one of the selected falling points and each of two or more of the remaining falling points included in a predetermined given range including the certain falling points, with respect to each of the selected falling points, thereby generating the falling frequency distribution by using the measured interval-times.

In this biological information processing apparatus, as a peak interval-time, for example, an interval-time between neighborhood peaks, i.e., an interval-time between peaks other than peaks laying side-by-side, is derived, so that it becomes possible to calculate the period from a more wide range of interval-times.

In yet another specific embodiment, in the above biological information processing apparatus, the frequency distribution generation section is operable: when generating the peak frequency distribution, to use a plurality of interval-times each measured from a respective one of a plurality of different combinations of two peaks; when generating the valley frequency distribution, to use a plurality of interval-times each measured from a respective one of a plurality of different combinations of two valleys; when generating the rising frequency distribution, to use a plurality of interval-times each measured from a respective one of a plurality of different combinations of two rising points; and when generating the falling frequency distribution, to use a plurality of interval-times each measured from a respective one of a plurality of different combinations of two falling points (for example, Steps 40 to 42).

In this biological information processing apparatus, an interval-time of the same combination, for example, of peaks, is never calculated plural times, so that it becomes possible to more accurately derive pulse rate.

In still another specific embodiment, in the above biological information processing apparatus, the period calculation section is operable to calculate at least two periods, respectively, from at least two frequency distributions generated by the frequency distribution generation section, and calculate, as the period of the first signal component, an average of selected two or more of the calculated periods, wherein two of the selected periods has a temporal difference falling within a predetermined range.

In this biological information processing apparatus, the period is determined from a plurality of types of frequency distributions of the interval-time, so that it becomes possible to more accurately derive pulse rate.

In yet still another specific embodiment, in the above biological information processing apparatus, the biological signal generation section is operable to generate a plurality of the biological signals based on a plurality of the estimates of the given third relationship, respectively; the second-order difference signal generation section is operable to generate a plurality of the second-order difference signals based on the biological signals, respectively; the frequency distribution generation section is operable to generate a plurality of the frequency distributions based on the second-order difference signals, respectively; and the period calculation section is operable to calculate respective periods of the second-order difference signals from the respective frequency distributions of the second-order difference signals, and calculate the period of the first signal component from the calculated periods.

In this biological information processing apparatus, the period is calculated from the plurality of second-order difference signals using the plurality of estimates of the third relationship, so that it becomes possible to derive pulse rate with less error.

In another further specific embodiment, in the above biological information processing apparatus, the period calculation section is operable to calculate an average of the calculated period of the first signal component and a previously-calculated period of the first signal component, as a new period of the first signal component, and, when the new period of the first signal component is deviated from a period calculated in the last measurement by a given time or more, to calculate an average of a period derived from the period calculated in the last measurement and a previously-calculated period, as another new period of the first signal component (for example, Steps S56 and S57).

In this biological information processing apparatus, the period is calculated additionally based on previously-derived period, so that it becomes possible to derive pulse rate with less error.

In still a further specific embodiment, in the above biological information processing apparatus, the frequency distribution generation section is operable to generate a weighted frequency distribution weighted with a weight based on a period previously calculated by the period calculation section

In an additional specific embodiment, in the above biological information processing apparatus, the frequency distribution generation section is operable to further generate a weighted frequency distribution weighted with a weight based on a period previously calculated by the period calculation section, and the period calculation section is operable, in the weighted frequency distribution and based on a given criteria, to determine an effective greatest weighted-frequency zone which is a zone which is a zone having a greatest frequency of the interval-time, and, based on an average time interval in the effective greatest weighted-frequency zone and the average time interval in the effective greatest frequency zone.

In this biological information processing apparatus, the period is calculated from a weighted frequency distribution weighted with a weight based on a previous calculation result, so that pulse rate is less likely to have a value fairly deviated from that of previously-derived pulse rate.

This application is based on Japanese Patent Application Serial No. 2012-40994 filed in Japan Patent Office on February 28, 2012, the contents of which are hereby incorporated by reference.

Although the present invention has been illustrated and described adequately and fully by way of example with reference to the accompanying drawings in order to represent the present invention, it is to be understood that various changes and modifications will be apparent to those skilled in the art. Therefore, unless otherwise such changes and modifications depart from the scope of the present invention hereinafter defined, they should be construed as being included therein.

Industrial Applicability

The present invention can provide a biological information processing apparatus and a signal processing method.

**Claims**

1. A biological information processing apparatus for, based on at least first measurement data and second measurement data obtained by emitting a plurality of light beams having respective different wavelengths to a living body and receiving corresponding light beams transmitted through or reflected by the living body, measuring biological information of the living body, comprising:

   a first measurement section configured to measure the first measurement data, the first measurement data comprising a first signal component having periodicity, and a first noise component;
   a second measurement section configured to measure the second measurement data, the second measurement data comprising a second signal component having a given first relationship with the first signal component, and a second noise component having a given second relationship with the first noise component;
   a biological signal generation section configured to generate a biological signal including the first signal component based on an estimate of a given third relationship, the first measurement data and the second measurement data;
   a difference signal generation section configured to generate a second-order difference signal by subjecting the biological signal to a second-order differencing operation;
   a frequency distribution generation section configured to generate at least one frequency distribution selected from the group comprising: a peak frequency distribution which represents a frequency distribution of an interval-time between peaks at each of which the second-order difference signal has a value equal to or greater than a given peak threshold; a valley frequency distribution which represents a frequency distribution of an interval-time between valleys at each of which the second-order difference signal has a value equal to or less than a given valley threshold; a rising frequency distribution which represents a frequency distribution of an interval-time between rising points at each of which a value of the second-order difference signal comes across a given threshold representing an intermediary value between the peaks and the valleys in a rising direction; and a falling frequency distribution which represents a frequency distribution of an interval-time between falling points at each of which a value of the second-order difference signal comes across the given threshold representing the intermediary value between the peaks and the valleys in a falling direction; and
   a period calculation section configured to, with respect to the frequency distribution generated by the frequency distribution generation section, determine an effective greatest frequency zone which is a zone having a greatest frequency of the interval-time, based on a given criterion, and calculate a period of the first signal component based on an average time interval in the effective greatest frequency zone.

2. The biological information processing apparatus as defined in claim 1, which further comprises:

   an estimation section configured to output respective estimates of an arterial blood absorption coefficient ratio associated with arterial blood and a venous blood absorption coefficient ratio associated with venous blood, each included in the first measurement data and the second measurement data; and
   a noise discrimination section configured to discriminate whether or not the second noise component largely includes a specific noise component due to blood having a value of oxygen saturation close to that of arterial blood,
   wherein the biological signal generation section is operable, when the noise discrimination section discriminates that the second noise component does not largely include the specific noise component, to determine the estimate of the given third relationship based on only the estimate of the venous blood absorption coefficient ratio, and, when the noise discrimination section discriminates that the second noise component largely includes the specific noise component, to determine, as the estimate of the given third relationship, a value between the respective estimates of the arterial blood absorption coefficient ratio and the venous blood absorption coefficient ratio.

3. The biological information processing apparatus as defined in claim 1 or 2, wherein the frequency distribution generation section is operable:

   when generating the peak frequency distribution in a given time range of the second-order difference signal, to select a plurality of peaks, and measure an interval-time between a certain one of the selected peaks and each of two or more of the remaining peaks included in a predetermined given range including the certain peak, with respect to each of the selected peaks, thereby generating the peak frequency distribution by using the measured interval-times;
   when generating the valley frequency distribution in a given time range of the second-order difference signal, to select a plurality of valley, and measure an interval-time between a certain one of the selected valley and each of two or more of the remaining valleys included in a predetermined given range including the certain valley, with respect to each of the selected valleys, thereby generating the valley frequency distribution by using the measured interval-times;

when generating the rising frequency distribution in a given time range of the second-order difference signal, to select a plurality of rising points, and measure an interval-time between a certain one of the selected rising points and each of two or more of the remaining rising points included in a predetermined given range including the certain rising points, with respect to each of the selected rising points, thereby generating the rising frequency distribution by using the measured interval-times; and

when generating the falling frequency distribution in a given time range of the second-order difference signal, to select a plurality of falling points, and measure an interval-time between a certain one of the selected falling points and each of two or more of the remaining falling points included in a predetermined given range including the certain falling points, with respect to each of the selected falling points, thereby generating the falling frequency distribution by using the measured interval-times.

4. The biological information processing apparatus as defined in any one of claims 1 to 3, wherein the frequency distribution generation section is operable:

when generating the peak frequency distribution, to use a plurality of interval-times each measured from a respective one of a plurality of different combinations of two peaks;
when generating the valley frequency distribution, to use a plurality of interval-times each measured from a respective one of a plurality of different combinations of two valleys;
when generating the rising frequency distribution, to use a plurality of interval-times each measured from a respective one of a plurality of different combinations of two rising points; and
when generating the falling frequency distribution, to use a plurality of interval-times each measured from a respective one of a plurality of different combinations of two falling points.

5. The biological information processing apparatus as defined in any one of claims 1 to 4, wherein the period calculation section is operable to calculate at least two periods, respectively, from at least two frequency distributions generated by the frequency distribution generation section, and calculate, as the period of the first signal component, an average of selected two or more of the calculated periods, two of the selected periods having a temporal difference falling within a predetermined range.

6. The biological information processing apparatus as defined in any one of claims 1 to 5, wherein:

the biological signal generation section is operable to generate a plurality of the biological signals based on a plurality of the estimates of the given third relationship, respectively;
the second-order difference signal generation section is operable to generate a plurality of the second-order difference signals based on the biological signals, respectively;
the frequency distribution generation section is operable to generate a plurality of the frequency distributions based on the second-order difference signals, respectively; and
the period calculation section is operable to calculate respective periods of the second-order difference signals from the respective frequency distributions of the second-order difference signals, and calculate the period of the first signal component from the calculated periods.

7. The biological information processing apparatus as defined in any one of claims 1 to 6, wherein the period calculation section is operable to calculate an average of the calculated period of the first signal component and a previously-calculated period of the first signal component, as a new period of the first signal component, and, when the new period of the first signal component is deviated from a period calculated in the last measurement by a given time or more, to calculate an average of a period derived from the period calculated in the last measurement and a previously-calculated period, as another new period of the first signal component.

8. The biological information processing apparatus as defined in any one of claims 1 to 7, wherein the frequency distribution generation section is operable to generate a weighted frequency distribution weighted with a weight based on a period previously calculated by the period calculation section.

9. The biological information processing apparatus as defined in any one of claims 1 to 7, wherein:

the frequency distribution generation section is operable to further generate a weighted frequency distribution weighted with a weight based on a period previously calculated by the period calculation section; and the period calculation section is operable, in the weighted frequency distribution and based on a given criteria, to determine an effective greatest weighted-frequency zone which is a zone which is a zone having a greatest frequency of the interval-time, and, based on an average time interval in the effective greatest weighted-frequency zone and the average time interval in the effective greatest frequency zone.

10. A signal processing method for use in a biological information processing apparatus configured to, based on at least first measurement data and second measurement data obtained by emitting a plurality of light beams having respective different wavelengths to a living body and receiving corresponding light beams transmitted through or reflected by the living body, measure biological information of the living body, wherein: the first measurement data comprises a first signal component having periodicity, and a first noise component; and the second measurement data comprises a second signal component having a given first relationship with the first signal component, and a second noise component having a given second relationship with the first noise component, the signal processing method comprising:

a biological signal generation step of generating a biological signal including the first signal component, based on an estimate of a given third relationship, the first measurement data and the second measurement data; a difference signal generation step of generating a second-order difference signal by subjecting the biological signal to a second-order differencing operation; a frequency distribution generation step of generating at least one frequency distribution selected from the group comprising: a peak frequency distribution which represents a frequency distribution of an interval-time between peaks at each of which the second-order difference signal has a value equal to or greater than a given peak threshold; a valley frequency distribution which represents a frequency distribution of an interval-time between valleys at each of which the second-order difference signal has a value equal to or less than a given valley threshold; a rising frequency distribution which represents a frequency distribution of an interval-time between rising points at each of which a value of the second-order difference signal comes across a given threshold representing an intermediary value between the peaks and the valleys in a rising direction; and a falling frequency distribution which represents a frequency distribution of an interval-time between falling points at each of which a value of the second-order difference signal comes across the given threshold representing the intermediary value between the peaks and the valleys in a falling direction; and a period calculation step of, with respect to the frequency distribution generated by the frequency distribution generation section, calculating a period of the first signal component based on the interval-time having a greatest frequency.

# FIG. 1

100

EP 2 821 008 A1

FIG. 2

EP 2 821 008 A1

FIG. 3

EP 2 821 008 A1

# FIG. 4

EP 2 821 008 A1

EP 2 821 008 A1

# FIG. 5

FIG. 6

EP 2 821 008 A1

# FIG. 7

FREQUENCY

VALLEY INTERVAL (SECOND)

LOCAL MAXIMUM REGION

LOCAL MAXIMUM REGION

EFFECTIVE GREATEST LOCAL MAXIMUM REGION

LOCAL MAXIMUM REGION

LOCAL MAXIMUM REGION

FIG. 8

FIG. 9

FIG. 10

```
        ┌─────────────────────────────────┐
        │  SUB-PROCESS OF DETERMINING     │ ── S21,22,23
        │  EFFECTIVE GREATEST FREQUENCY   │
        │  ZONES AND EFFECTIVE GREATEST   │
        │  WEIGHTED-FREQUENCY ZONES       │
        └─────────────────────────────────┘
                        │
                        ▼
  ┌──────────────────────────────────────────┐ ── S10
  │  DETERMINATION OF LOCAL MAXIMUM ZONE REGIONS │
  └──────────────────────────────────────────┘
                        │
                        ▼
  ┌──────────────────────────────────────────┐ ── S11
  │  FOR EACH LOCAL MAXIMUM ZONE REGION,       │
  │  CALCULATING LOCAL MAXIMUM FREQUENCY,      │
  │  REGION WIDTH, AND LOCAL AVERAGE INTERVAL-TIME │
  └──────────────────────────────────────────┘
                        │
                        ▼
  ┌──────────────────────────────────────────┐ ── S12
  │  DETERMINATION OF EFFECTIVE GREATEST FREQUENCY ZONE │
  └──────────────────────────────────────────┘
                        │
                        ▼
  ┌──────────────────────────────────────────┐ ── S13
  │  DETERMINATION OF WEIGHTING FACTOR FOR     │
  │  EACH LOCAL MAXIMUM ZONE REGION            │
  └──────────────────────────────────────────┘
                        │
                        ▼
  ┌──────────────────────────────────────────┐ ── S14
  │  CALCULATION OF TEMPORARY WEIGHTED LOCAL MAXIMUM │
  │  FREQUENCY IN EACH LOCAL MAXIMUM ZONE REGION │
  └──────────────────────────────────────────┘
                        │
                        ▼
                   ◇ REGION WIDTH ≧ 4 ◇  ── S15 ──── NO
                        │ YES
                        ▼
  ┌──────────────────────────────────────────┐ ── S16
  │  DETERMINATION OF WEIGHTED LOCAL MAXIMUM FREQUENCY │
  └──────────────────────────────────────────┘
                        │
                        ▼
           ◇ HAVE ALL LOCAL MAXIMUM ZONE        ◇ ── S17
     NO ── ◇ REGIONS BEEN SUBJECTED TO RE-CALCULATION ◇
           ◇ PROCESSING ?                       ◇
                        │ YES
                        ▼
  ┌──────────────────────────────────────────┐ ── S18
  │  DETERMINATION OF EFFECTIVE GREATEST       │
  │  WEIGHTED-FREQUENCY ZONE                   │
  └──────────────────────────────────────────┘
                        │
                        ▼
                 ┌──────────────┐
                 │  END OF      │
                 │  SUB-PROCESS │
                 └──────────────┘
```

# FIG. 11

PERIOD CALCULATION PROCESS ⟶ S47

↓

CREATION OF FREQUENCY DISTRIBUTIONS OF PEAK, VALLEY AND RISING ⟶ S20

↓

REGARDING PEAK INTERVAL FREQUENCY DISTRIBUTION, DETERMINING EFFECTIVE MAXIMUM FREQUENCY ZONE AND EFFECTIVE MAXIMUM WEIGHTED-FREQUENCY ZONE ⟶ S21

↓

REGARDING VALLEY INTERVAL FREQUENCY DISTRIBUTION, DETERMINING EFFECTIVE MAXIMUM FREQUENCY ZONE AND EFFECTIVE MAXIMUM WEIGHTED-FREQUENCY ZONE ⟶ S22

↓

REGARDING RISING INTERVAL FREQUENCY DISTRIBUTION, DETERMINING EFFECTIVE MAXIMUM FREQUENCY ZONE AND EFFECTIVE MAXIMUM WEIGHTED-FREQUENCY ZONE ⟶ S23

↓

CALCULATION OF temp_period_peak (AVERAGE OF PEAK INTERVALS IN MAXIMUM FREQUENCY ZONE) ⟶ S24

↓

CALCULATION OF temp_period_valley (AVERAGE OF VALLEY INTERVALS IN MAXIMUM FREQUENCY ZONE) ⟶ S25

↓

CALCULATION OF temp_period_rising (AVERAGE OF RISING INTERVALS IN MAXIMUM FREQUENCY ZONE) ⟶ S26

↓

①

FIG. 12

① 

↓

S27

◇ flg_low_noise = 1?
(IS ARTERIAL BLOOD NOISE
LARGE?) ◇ —— NO ——→

YES ↓

S28

◇ ARE
temp_period_peak,
temp_period_valley AND
temp_period_rising IN APPROXIMATE
RELATION TO EACH
OTHER
? ◇ —— NO ——→

YES ↓

S29

┌──────────────────────────┐
│ CALCULATION OF AVERAGE   │
│ OF temp_period_peak,     │
│ temp_period_valley AND   │
│ temp_period_rising       │
│ (TEMPORARY PERIOD)       │
└──────────────────────────┘

↓

S30

┌──────────────────────────┐
│ CALCULATION FLAG         │
│ flg_same = 1             │
└──────────────────────────┘

S31

◇ IS
ONLY AT
LEAST ONE OF
temp_period_valley AND
temp_period_rising IN APPROXIMATE
RELATION TO
temp_period
_peak
? ◇ —— NO ——→

YES ↓

S32

┌──────────────────────────┐
│ CALCULATION OF AVERAGE   │
│ OF temp_period_peak AND  │
│ EITHER ONE OF            │
│ temp_period_valley       │
│ AND temp_period_rising   │
│ (TEMPORARY PERIOD)       │
└──────────────────────────┘

↓

S33

┌──────────────────────────┐
│ CALCULATION FLAG         │
│ flg_same = 1             │
└──────────────────────────┘

S34

┌────────────────────────────────┐
│ CALCULATING: FREQUENCY        │
│ (max_freq_weighted) IN ONE ZONE│
│ HAVING LARGEST FREQUENCY      │
│ AMONG EFFECTIVE MAXIMUM       │
│ WEIGHTED-FREQUENCY            │
│ ZONES FOR PEAK INTERVAL,      │
│ VALLEY INTERVAL AND RISING    │
│ INTERVAL ; AND AVERAGE        │
│ INTERVAL (TEMPORARY WEIGHTED  │
│ PERIOD temp_period_weighted)  │
│ IN THE ONE ZONE               │
└────────────────────────────────┘

↓

( END OF PROCESS )

# FIG. 13

PULSE RATE CALCULATION PROCESS

S40 — flg_low_noise = 1?
(IS ARTERIAL BLOOD NOISE LARGE?)

NO → S42 — p = pv_es

YES → S41 — p = pa_es × 1.3

S43 — CALCULATION OF [R − p × IR]

S44 — CALCULATION OF SECOND-ORDER DIFFERENCE OF [R − p × IR]

S45 — DETERMINATION OF THRESHOLDS

S46 — DETECTION OF PEAK, VALLEY AND RISING

S47 — PERIOD CALCULATION PROCESS (CALCULATION OF temp_period)

S48 — flg_low_noise = 1?

NO

YES → S49 — HAS THREE CYCLES BEEN EXECUTED?

S50 — IS NEXT CYCLE IS 2ND CYCLE OR 3RD CYCLE?

3RD CYCLE

2ND CYCLE → S51 — p = pa_es × 1.5

S52 — p = pa_es × 1.1

YES → S53 — ARE ONE OR MORE OF CALCULATION FLAGS flg_same FOR THREE VALUES OF P SET TO 1?

NO → S55 — CALCULATING temp_period_weighted CORRESPONDING TO LARGEST temp_freq_weighted, AS temp_ave_period

YES → S54 — CALCULATION OF AVERAGE OF TEMPORARY PERIODS temp_period (temp_ave_period)

S56 — A DIFFERENCE BETWEEN temp_ave_period AND PREVIOUS ave_period?

NO

YES → S57 — ADJUSTMENT OF temp_ave_period

S58 — CALCULATING AVERAGE OF TEMPORAL AVERAGE PERIOD temp_ave_period IN THIS TIME AND GIVEN NUMBER OF PREVIOUSLY CALCULATED TEMPORAL AVERAGE PERIODS temp_ave_period, AS ave_period IN THIS TIME

S59 — ave_PR = 60 / ave_period

END OF PROCESS

FIG. 14

$\big($SpO2 AND PULSE RATE MEASURING PROCESS$\big)$

S70

ACQUISITION OF PULSE
WAVE SIGNALS R(i), IR(i)

S71

CALCULATION OF pv_es AND pa_es

S72

CALCULATION OF VENOUS (ARTERIAL?)
BLOOD NOISE INDEX

S73

IS
ARTERIAL BLOOD
NOISE INDEX GREATER THAN
PREDETERMINED
VALUE?

NO

YES

S74

flg_low_noise = 1

S75

flg_low_noise = 0

S76

CALCULATION OF SpO2 BASED ON pa_es

S77

STORAGE OF CALCULATED SpO2

S78

CALCULATING AVERAGE av_SpO2 OF
CALCULATED SpO2 AND PREVIOUS SpO2

S79

PULSE RATE (ave_PR)
CALCULATION PROCESSING

S80

INDICATION OF ave_SpO2 AND ave_PR

# FIG. 15

EP 2 821 008 A1

| **INTERNATIONAL SEARCH REPORT** | International application No. |
|---|---|
| | PCT/JP2013/000807 |

| A. CLASSIFICATION OF SUBJECT MATTER |
|---|
| *A61B5/0245*(2006.01)i, *A61B5/1455*(2006.01)i |

According to International Patent Classification (IPC) or to both national classification and IPC

| B. FIELDS SEARCHED |
|---|
| Minimum documentation searched (classification system followed by classification symbols) |
| A61B5/0245, A61B5/1455 |

Documentation searched other than minimum documentation to the extent that such documents are included in the fields searched

| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 2011-206285 A (Konica Minolta Sensing, Inc.), 20 October 2011 (20.10.2011), entire text; all drawings (Family: none) | 1-10 |
| A | JP 2009-297184 A (Fujitsu Ltd.), 24 December 2009 (24.12.2009), entire text; all drawings & US 2009/0312647 A1 | 1-10 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 March, 2013 (12.03.13) | 19 March, 2013 (19.03.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010073908 A

- JP 2012040994 A